# EUROPEAN PATENT SPECIFICATION

(11) **EP 0 642 588 B1**
(45) Date of publication and mention of the grant of the patent: **14.08.2002**
(21) Application number: 93910462.6
(22) Date of filing: 18.05.1993
(51) Int. Cl.: C12Q 1/68

(54) **METHODS FOR THE DIAGNOSIS OF FUNGAL INFECTIONS**
METHODEN ZUR DIAGNOSE VON PILZINFEKTIONEN
PROCEDES PERMETTANT DE DIAGNOSTIQUER DES MYCOSES

(30) Priority: 18.05.1992 NZ 24278392
(43) Date of publication of application: 15.03.1995
(60) Divisional application: 02076305.8
(73) Proprietor: HOLMES, Ann Rachel, Dunedin 9001 (NZ); CANNON, Richard David, Dunedin 9001 (NZ); JENKINSON, Howard Francis, Dunendin 9001 (NZ); SHEPHERD, Maxwell Gilbert, Dunedin 9001 (NZ)
(72) Inventor: HOLMES, Ann Rachel, Dunedin 9001 (NZ); CANNON, Richard David, Dunedin 9001 (NZ); JENKINSON, Howard Francis, Dunendin 9001 (NZ); SHEPHERD, Maxwell Gilbert, Dunedin 9001 (NZ)
(74) Representative: Thomas, Philip John Duval
(86) International application number: NZ9300039
(87) International publication number: WO9323568

(56) References cited:
- EP-A- 0 175 283
- EP-A- 0 335 633
- AU-A- 6 390 590
- WHITE T ET AL: "AMPLIFICATION AND DIRECT SEQUENCING OF FUNGAL RIBOSOMAL RNA GENES FOR PHYLOGENETICS" PCR PROTOCOLS, GUIDE TO METHODS AND APPLICATIONS, 1990, INNIS M A;GELFAND D H; SNINSKY J J; WHITE T J, pages 315-322, XP002017490
- VERMA M ET AL: "Phylogenetic implications of heterogeneity of the non transcribed spacer of rDNA repeating unit of various Neurospora and related fungal species" CURRENT GENETICS, vol. 11, 1987, pages 309-14, XP002055163
- KAZUHIRO KITADA ET AL: 'Detection of Pneumocystic Carinii Sequences by Polymerase Chain Reaction: Animal Models and Clinical Application to Noninvasive Specimens' JOURNAL OF CLINICAL MICROBIOLOGY vol. 29, no. 9, pages 1985 - 1990, XP000198172
- CHEN MIN WEI ET AL. NUCLEIC ACIDS RESEARCH vol. 12, no. 12, 1984, pages 4881 - 4892, XP002940612

## Description

### TECHNICAL FIELD OF THE INVENTION

This invention relates generally to methods of detecting the presence or absence of human pathogenic yeasts and other pathogenic fungi in a sample such as blood or human tissue. It further relates to nucleic acid probes suitable for use in such methods as well as to diagnostic kits incorporating such probes.

### BACKGROUND OF THE INVENTION

There are almost 200 species in the diverse fungal genus of *Candida,* eight of which infect humans, these being *Candida albicans, Candida tropicalis, Candida glabrata, Candida kefyr, Candida parapsilosis, Candida krusei, Candida guilliermondii and Candida lusitaniae*. These species can become opportunistic human pathogens when host defences are lowered and they are the most frequently observed fungal infections of the immunocompromised.

*Candida* species are usually commensal but also may cause a wide spectrum of disease, from oral and vaginal mucosal thrush, to severe mucocutaneous candidosis in AIDS patients, to invasive disseminated infection in post-operative and immunocompromised patients. Further, data from the United States National Nosocomial Infections Surveillance system shows that the occurrence of life threatening invasive *Candida* infection is increasing (Edwards J E, N Eng J Med 324 1060-2 (1991). Unfortunately, many patients die with undiagnosed invasive *Candida* infections and one US study (Wey S B *et al*., Arch Intern Med 2642-1645 (1988)) has reported an attributable mortality rate to *Candida* of 26-49%. This high attributable mortality rate is due to the considerable susceptibility of certain at-risk groups, such as leukaemia patients.

Treatment of invasive *Candida* infections is often delayed until the causative organism is identified because the antifungal drugs used have host toxicity, yet there is a much improved prognosis if antifungal therapy is prompt. The focus of any treatment regime is therefore on the specificity and rapidity of diagnosis of the infection.

There is at present no single criterion for diagnosis of disseminated candidosis. Diagnosis must be based on the clinical assessment of a number of indications, both from laboratory findings and the patient's condition. Cumulative information currently used for diagnosis includes fever with failure to respond to antibacterial agents. presence of eye and skin lesions, positive blood cultures, recovery of yeasts from biopsies and laboratory data from serological tests including antigen detection. Furthermore, while tests for circulating antigen are available, recent studies (Phillips P *et al*., J Clin Microbiol 28 2320-6 (1990), Walsh T J *et al*., N Eng J Med 324 1026-31 (1991)) have indicated limitations in sensitivity and specificity compared to blood culture.

All of the above clinical indicators take time to assess. Even using an optimal blood culture system such as the lysis centrifugation method (Dorn G L *et al.,* J Clin Microbiol 7 52-4 (1978), Jones J M, Clin Microbiol Rev 3 32-45 (1990)), the detection of candidaemia takes a minimum of two days. There is accordingly a need for a rapid, sensitive and specific test to aid in the diagnosis of the infections such as candidaemia caused by pathogenic yeasts.

Kirada et al., J. Clin Microbiol 29 1985-90 (1991) describes a PCR-based method for detecting Pneumocystis carini in a sample iniding the amplification and detection (by ethidium bromide staining) of 5S rDNA. Vema and Dutta Curr. Genet. 11 309-14 (1987) describes hybridization studies in which non-conserved, non-transcribed spacer regions (NB) of rRNA genes of fungal species are used to determine phylogenetic relatedness. The utility of rRNA sequences for phylogenetic analyses is also described in white et al, In "PCR Protocols: a guide to methods and applications" (1990), Academic Press, pages 3,5-20.

It is accordingly an object of the present invention to provide a method or methods for the detection of fungal infections which is specific, sensitive and rapid, or at least to provide the public with a useful choice.

### SUMMARY OF THE INVENTION

Accordingly, in a first aspect, described is a method of detecting the presence of one or more of a group of target fungal organisms in a sample comprising the step of testing said sample to detect the presence or absence of part or all of a double-stranded DNA molecule characteristic of and specific for said organisms, said DNA molecule comprising a nucleotide sequence coding for the 5S rRNA gene of one of said organisms.

Preferably, said sample contains DNA extracted from blood or tissue.

Conveniently, the target organisms are selected from the group consisting of members of the genus *Candida* and *Saccharomyces cerevisiae.*

In a preferred embodiment, the testing step comprises:
(i) amplifying any DNA present in said sample having a nucleotide sequence of part or all of the 5S rRNA gene of a target organism;
(ii) detecting the presence or absence of said amplified DNA in said sample.

Suitably, the amplification procedure employed is the polymerase chain reaction, the ligase detection reaction or the ligase chain reaction.

Alternatively, said testing step includes the sub-steps of:
(i) testing said sample with an optionally labelled nucleic acid probe capable of hybridising to part or all of one strand of said DNA molecule, said testing being performed under conditions such that said probe will hybridise to any DNA coding for the 5S rRNA gene of any target organism which is present in the sample to form hybrid DNA;
(ii) detecting the presence or absence of said hybrid DNA in the sample.

In this embodiment, it is further preferred that the testing be performed under low stringency hybridisation conditions.

In a preferred embodiment, the nucleic acid probe has a nucleotide sequence comprising part or all of the nucleotide sequence from nucleotide 937 to nucleotide 1057 of SEQ ID No 1.

In a further preferred embodiment, the nucleic acid probe consists of a 0.4 kb *Pst*I/*Bam*H1 digestion fragment derived from the rRNA repeat unit DNA of *C. albicans* strain 10261 (ATCC 10261), a sub-fragment thereof, or has a nucleotide sequence which hybridises at high stringency thereto.

Suitably, said nucleic acid probe is labelled.

In a further aspect, described is a method of detecting the presence of a target fungal organism in a sample comprising the step of testing said sample to detect the presence or absence of part or all of a double-stranded DNA molecule characteristic of and specific for said organism, said DNA molecule comprising a nucleotide sequence containing a non-transcribed spacer region of the rRNA repeat unit of said organism.

Preferably, said sample contains DNA extracted from blood or tissue.

Conveniently, the target organism is a yeast which is a member of the genus *Candida* or is *Saccharomyces cerevisiae*.

More conveniently, the target organism is *Candida albicans.*

In a preferred embodiment, the testing step comprises:
(i) amplifying any DNA present in said sample having a nucleotide sequence of part or all of a non-transcribed spacer region of the rRNA repeat unit of the target organism;
(ii) detecting the presence or absence of said amplified DNA in said sample.

Suitably, the amplification procedure employed is the polymerase chain reaction, the ligase detection reaction or the ligase chain reaction.

Alternatively, the testing step includes the sub-steps of:
(i) testing said sample with an optionally labelled nucleic acid probe capable of hybridising to part or all of one strand of said DNA molecule, said testing being performed under conditions such that said probe will hybridise to any DNA containing a non-transcribed spacer region of the rRNA repeat unit of the target organism which is present in the sample to form hybrid DNA;
(ii) detecting the presence or absence of said hybrid DNA in the sample.

In this embodiment, it is further preferred that the testing be performed under low stringency hybridisation conditions.

In a preferred embodiment, the target organism is *C. albicans* and the nucleic acid probe has a nucleotide sequence comprising part or all of the nucleotide sequence from nucleotide 1 to nucleotide 936 of SEQ ID No 1.

In a further preferred embodiment, the target organism is *C. albicans* and the nucleic acid probe consists of a 0.9 kb *Pst*I digestion fragment derived from the rRNA repeat unit of *C. albicans* strain 10261 (ATCC 10261), a sub-fragment thereof, or has a nucleotide sequence which hybridises at high stringency thereto.

Suitably, said nucleic acid probe is labelled.

In yet a further aspect, described is a method of detecting the presence of one or more of a group of target fungal organisms in a sample comprising the steps of:
(a) testing said sample to detect the presence of part or all of a double-stranded DNA molecule characteristic of and specific for said organisms, said DNA molecule comprising a nucleotide sequence coding for the 5S rRNA gene of one of said organisms; and
(b) separately testing said sample to detect the presence of part or all of one or more of a group of double-stranded DNA molecules, each said double-stranded DNA molecule being characteristic of and specific for a different target organism and comprising a nucleotide sequence of a non-transcribed spacer region of the rRNA repeat unit of that organism.

Conveniently, testing step (a) comprises the sub-steps of:
(i) amplifying any DNA present in said sample having a nucleotide sequence of the 5S rRNA gene of a target organism; and
(ii) detecting the presence or absence of any amplified DNA in said sample,
and testing step (b) comprises the sub-steps of:
(i) amplifying any of said group of DNA molecules present in said sample; and
(ii) detecting the presence or absence of any of said group of DNA molecules in amplified quantities.

Preferably, the amplification procedure employed is the polymerase chain reaction.

In the alternative, testing step (a) comprises the sub-steps of:
(i) testing said sample with a labelled nucleic acid probe capable of hybridising to part or all of one strand of a double-stranded DNA molecule characteristic of and specific for said target organisms, said DNA molecule comprising a nucleotide sequence coding for the 5S rRNA gene of one of said organisms, said testing being performed under conditions such that said probe will hybridise to any DNA coding for the 5S rRNA gene of any of the target organisms present in the sample to form labelled hybrid DNA; and
(ii) detecting any labelled hybrid DNA in said sample, '
and testing step (b) comprises the sub-steps of:
(i) testing said sample with at least one labelled nucleic acid probe, each probe carrying a different label and being capable of hybridising to part or all of one strand of a double-stranded DNA molecule characteristic of and specific for a different target organism, said DNA molecule comprising a nucleotide sequence containing a non-transcribed spacer region of the rRNA repeat unit of that target organism, said testing being performed under conditions such that each said probe will hybridise to any DNA containing the non-transcribed spacer region of the rRNA repeat unit of its target organism to form labelled hybrid DNA; and
(ii) detecting any labelled hybrid DNA in said sample.

In still a further aspect, described is a method of detecting and identifying one or more of a group of target fungal organisms in a sample which may contain said organisms comprising the steps of:
(i) testing said sample
   (a) with a primary labelled nucleic acid probe capable of hybridising to part or all of one strand of a double-stranded DNA molecule characteristic of and specific for said target organisms, said DNA molecule comprising a nucleotide sequence coding for the 5S rRNA gene of one of said organisms, said testing being performed under conditions such that said probe will hybridise to any DNA coding for the 5S rRNA gene of any of the target organisms present in the sample to form labelled hybrid DNA; and
   (b) with at least one secondary labelled nucleic acid probe, each probe carrying a different label and being capable of hybridising to part or all of one strand of a double-stranded DNA molecule characteristic of and specific for a different target organism, said DNA molecule comprising a nucleotide sequence containing a non-transcribed spacer region of the rRNA repeat unit of that target organism, said testing being performed under conditions such that each said probe will hybridise to any DNA containing the non-transcribed spacer region of the rRNA repeat unit of its target organism to form labelled hybrid DNA;
(ii) detecting and identifying the target organism(s) present with reference to the differential labels of said hybrid DNA(s) in said sample.

Preferably, the target organisms are selected from the group consisting of members of the genus *Candida* and *Saccharomyces cerevisiae.*

Conveniently, the detection step includes the addition of an amount of a DNA ligase to said sample sufficient to ligate any primary and secondary probes hybridised to adjacent regions of a DNA strand.

In a preferred embodiment, said primary nucleic acid probe is capable of hybridising to part or all of the 5S rRNA gene of *C. albicans.*

In a further preferred embodiment, said primary nucleic acid probe is capable of hybridising to part or all of the nucleotide sequence from nucleotide 937 to nucleotide 1057 of SEQ ID No 1.

In still a further preferred embodiment, said primary nucleic acid probe is a labelled 0.4 kb *Pst*I/*Bam*H1 digestion fragment derived from the rRNA repeat unit of *C. albicans* strain 10261 (ATCC 10261), a sub-fragment thereof, or has a nucleotide sequence which hybridises at high stringency thereto.

Preferably, one said secondary nucleic acid probe is specific for *C. albicans* and capable of hybridising to part or all of the non-transcribed spacer region of the rRNA repeat unit of *C. albicans.*

In the alternative, one said secondary nucleic acid probe is specific for *C. albicans* and is capable of hybridising to part or all of the nucleotide sequence from nucleotide 1 to nucleotide 936 of SEQ ID No 1.

In still a further alternative, one said secondary nucleic acid probe is specific for *C. albicans* and is a labelled 0.9 kb *Pst*I digestion fragment derived from the rRNA repeat unit of *C. albicans* strain 10261 (ATCC 10261), a sub-fragment thereof, or has a nucleotide sequence which hybridises at high stringency thereto.

Conveniently, other said secondary nucleic acid probe(s) are capable of hybridising to part or all of a non-transcribed spacer region of the rRNA repeat unit of one or more of *C. tropicalis, C. glabrata, C. kefyr, C. parapsilosis, C. krusei, C. guilliermondii, C. lusitaniae and S. cerevisiae.*

In a further aspect, described is an optionally labelled nucleic acid probe capable of hybridising to part or all of one strand of a double-stranded DNA molecule characteristic of and specific for a group of target fungal organisms, said DNA molecule comprising a nucleotide sequence coding for the 5S rRNA gene of one said organism.

Preferably, the target organisms are selected from the group consisting of members of the genus *Candida* and *Saccharomyces cerevisiae.*

In one embodiment, the nucleotide sequence to which the probe is capable of hybridising codes for the 5S rRNA gene of *C. albicans.*

In an alternative embodiment, the nucleotide sequence to which the probe is capable of hybridising is from nucleotide 937 to nucleotide 1057 of SEQ ID No 1.

Conveniently, the probe is an oligonucleotide of at least 14 nucleotides in length.

Alternatively, the probe comprises at least 20 consecutive nucleotides from the nucleotide sequence between nucleotide 937 and nucleotide 1057 of SEQ ID No 1.

As a still further alternative, the probe is a 0.4 kb *Pst*I/*Bam*H1 digestion fragment derived from the rRNA repeat unit of *C. albicans* strain 10261 (ATCC 10261), a sub-fragment thereof, or has a nucleotide sequence which hybridises at high stringency thereto.

In yet a further aspect, described is an optionally labelled nucleic acid probe capable of hybridising to part or all of one strand of a double-stranded DNA molecule characteristic of and specific for a single target fungal organism, said DNA molecule comprising a nucleotide sequence containing a non-transcribed spacer region of the rRNA repeat unit of said organism.

Conveniently, the target organism is a yeast which is a member of the genus *Candida* or is *Saccharomyces cerevisiae.*

In a preferred embodiment, the target organism is *C. albicans* and the nucleotide sequence to which the probe is capable of hybridising is that shown from nucleotide 1 to nucleotide 936 of SEQ ID No 1.

In one embodiment, the probe is an oligonucleotide of at least 14 nucleotides in length.

In a further embodiment, the probe comprises at least 20 consecutive nucleotides from the nucleotide sequence between nucleotide 1 and nucleotide 936 of SEQ ID No 1.

In still a further embodiment, the probe is a 0.9 kb *Pst*I digestion fragment derived from the rRNA repeat unit of *C. albicans* strain 10261 (ATCC 10261), a sub-fragment thereof, or has a nucleotide sequence which hybridises at high stringency thereto.

In still a further aspect, described is a diagnostic kit for use in detecting the presence of a target fungal organism in a sample which includes an optionally labelled nucleic acid probe capable of hybridising to part or all of one strand of a double-stranded DNA molecule characteristic of and specific for said organism, said DNA molecule comprising a nucleotide sequence containing a non-transcribed spacer region of the rRNA repeat unit of said organism.

In yet a further aspect, described is a diagnostic kit for use in detecting the presence of one or more of a group of target fungal organisms in a sample which includes an optionally labelled nucleic acid probe capable of hybridising to part or all of one strand of a double-stranded DNA molecule characteristic of and specific for said organisms, said DNA molecule comprising a nucleotide sequence coding for the 5S rRNA gene of one of said organisms.

In a further aspect, described is a diagnostic kit for use in detecting and identifying one or more of a group of target fungal organisms in a sample, said kit including:
(i) a primary labelled nucleic acid probe capable of hybridising to part or all of one strand of a double-stranded DNA molecule characteristic of and specific for said target organisms, said DNA molecule comprising a nucleotide sequence coding for the 5S rRNA gene of one of said organisms;
(ii) at least one secondary labelled nucleic acid probe, each probe carrying a different label and being capable of hybridising to part or all of one strand of a double-stranded DNA molecule characteristic of and specific for a different target organism, said DNA molecule comprising a nucleotide sequence containing the non-transcribed spacer region of the rRNA repeat unit of that target organism.

In still a further aspect, described is a diagnostic kit for use in detecting the presence of one or more of a group of target fungal organisms in a sample which includes optionally labelled paired nucleic acid primers which are capable of binding to, and which define, part or all of a double-stranded DNA molecule characteristic of and specific for said organisms, said DNA molecule comprising a nucleotide sequence coding for the 5S rRNA gene of one of said organisms.

As yet a further aspect, described is a diagnostic kit for use in detecting the presence of a target fungal organism in a sample which includes optionally labelled paired nucleic acid primers which are capable of binding to, and which define, part or all of a double-stranded DNA molecule characteristic of and specific for said organism, said DNA molecule comprising a nucleotide sequence containing a non-transcribed spacer region of the rRNA repeat unit of said organism.

As another aspect, described is a diagnostic kit for use in detecting and identifying one or more of a group of target fungal organisms in a sample, said kit including:
(i) optionally labelled paired nucleic acid primers which are capable of binding to, and which define, part or all of a double-stranded DNA molecule characteristic of and specific for said organisms, said DNA molecule comprising a nucleotide sequence coding for the 5S rRNA gene of one of said organisms; and
(ii) optionally labelled paired nucleic acid primers for each of one or more target organisms, the pairs of primers each defining part or all of a double-stranded DNA molecule characteristic of and specific for a particular target organism, said DNA molecule comprising a nucleotide sequence coding for a non-transcribed spacer region of the rRNA repeat unit of that organism.

In still a further aspect, described is a method of treating fungal cells containing fungal DNA to make such DNA available for detection or amplification comprising the steps of:
centrifuging a receptacle containing a solution of fungal cells and a density gradient centrifugation medium incorporating a cell wall disrupting agent, said centrifugation being performed under conditions such that at least the major proportion of fungal cells move out of solution into said centrifugation medium, resulting in the formation of a supernatant and a centrifugation medium containing fungal cells and said cell wall disrupting agent;
separating said supernatant and said centrifugation medium;
incubating said centrifugation medium for a period of time and at a temperature sufficient for said cell wall disrupting agent to disrupt the cell wall of the fungal cells present in said medium to form fungal cell protoplasts;
admixing said incubated centrifugation medium containing fungal cell protoplasts and an amount of a chelating resin; and
heating said admixture for a period of time and at a temperature sufficient to lyse said protoplasts and to release fungal DNA in a denatured form.

Preferably, said released fungal DNA is then recovered.

### SUMMARY OF THE DRAWINGS

While the present invention is broadly as defined above, it will be appreciated by those persons skilled in the art that it is not limited thereto but that it includes embodiments of which the following description provides examples. Moreover, a better understanding of the present invention will be gained from reference to the accompanying drawings in which
Figure 1 shows the nucleotide sequence of 1.2 kb *C. albicans* 10261 DNA containing both the 5S rRNA gene (enclosed in heavy box) and the non-transcribed spacer region of the *C. albicans* rRNA DNA repeat unit. The DNA was sequenced using the dideoxy chain termination method of Sanger *et al*., Proc Natl Acad Sci USA 74 5463-5467 (1977)). Certain restriction sites are boxed and labelled;

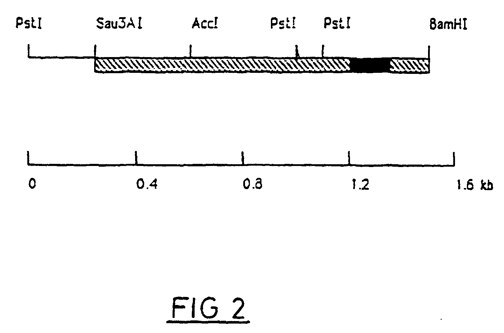

Figure 2 is a restriction enzyme digestion map of *C. albicans* 10261 DNA fragment including the sequence (hatched area) shown in Figure 1. The 5S rRNA gene is indicated by the solid area;

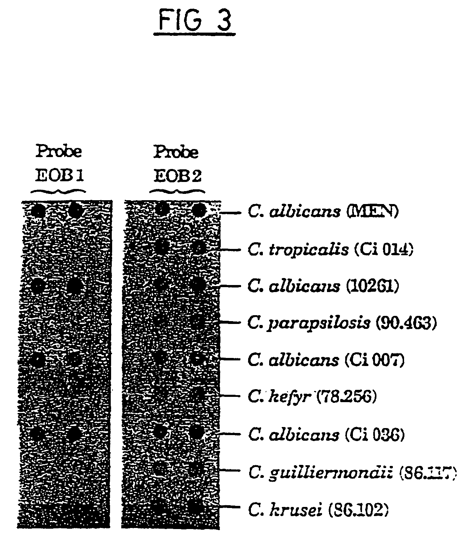

Figure 3 shows the specifics of ³⁵S dATP labelled probes EOB1 and EOB2 for *Candida* DNA;
Figure 4 shows the sensitivities of ³⁵S dATP labelled probes EOB1 and EOB2.
Figure 5 is a schematic diagram showing the orientation and position of oligonucleotide primers used in PCR amplification of both the conserved and species-specific regions of the rRNA DNA.
Figure 6 is a representative ethidium bromide stained agarose gel and a Southern blot thereof showing the PCR products obtained using different *C. albicans* strains and other DNAs as target DNA and EOB1 primers (Example 5).
Figure 7 is a representative ethidium bromide stained agarose gel showing the PCR products obtained in "semi-nested" and multiplex PCRs (Example 6).

### DETAILED DESCRIPTION OF THE INVENTION

The present invention is based upon the applicants' investigations in relation to the rRNA repeat unit of pathogenic yeasts, including pathogenic yeasts selected from the genus *Candida* and from *S. cerevisiae.*

The rRNA repeat unit is a tandem repeat region of DNA which codes for the ribosomal RNAs as well as containing non-transcribed spacer regions. In *S. cerevisiae* there are 100-150 copies of the rRNA genes and they form a single tandem array on chromosome XII with a repeat length of approximately 9 kb (Petes T D, Proc Natl Acad Sci USA 76 410-414 (1979); Bell G I *et al*., J Biol Chem 252 8118-8125 (1977)). In *C. albicans* and in other *Candida* species the arrangement of the tandem repeats is very similar (Magee *et al.,* J Bacteriol 169 1639-1643 (1987)).

It has now been determined by the applicants that the rRNA repeat unit of pathogenic fungi contains portions of DNA which are totally distinct in terms of specificity for the fungus in question. The first of these portions of DNA is that coding for the 5S rRNA gene which the applicants have found varies only slightly between fungi. This region is therefore specific for pathogenic fungi in general (i.e. capable of distinguishing between fungi and other organisms) while at the same time being generally non-specific as to the identity of the pathogenic fungus involved (i.e. not generally capable of distinguishing between the various pathogenic fungi).

In total contrast, a second portion of the rRNA repeat unit of pathogenic fungi has been found to be specific for a particular fungal species (i.e. capable of distinguishing between that fungal species and all other organisms). This portion is the non-transcribed spacer region (or regions) of the rRNA repeat unit. Indeed, to date the applicants have shown that the non-transcribed spacer regions of numerous fungal organisms including *C. albicans, C. tropicalis, C. krusei, C. parapsilosis, C. kefyr, C. guilliermondii* and *S. cerevisiae* do not hybridise under standard laboratory hybridisation conditions.

It is broadly upon the applicants' findings in relation to the specific and non-specific regions of the rRNA repeat unit of pathogenic fungi that the present invention is based.

Accordingly, described are methods and means for the detection of fungal organisms in a sample. This sample can be any medium which may contain the fungus itself but will usually be a sample which contains DNA extracted from blood or tissue, particularly human blood or tissue.

In its first general aspect, described are methods for the detection of fungal organisms in the sample. The methods described each require the detection of the presence in the sample of DNA characteristic of the target organism or organisms. This detection may be by a number of methods.

A first and preferred method involves the use of probes which comprise nucleic acid capable of hybridising to a preselected region of the rRNA repeat unit. This preselected region will depend upon whether the method is to be used as a general screening assay employed in relation to a large number of samples to detect the presence of any pathogenic fungus, or whether the method is to be used to specifically identify a particular fungal organism as being present in the sample.

Where the method is to be used as general screening assay, the probe will comprise nucleic acid capable of hybridising to part or all of one strand of a double-stranded DNA molecule coding for the 5S rRNA gene of any one of the target fungal organisms. For example, the probe can be hybridisable to the 5S rRNA gene of an organism which is a member of the genus *Candida* (such as *Candida albicans, Candida tropicalis, Candida glabrata, Candida kefyr, Candida parapsilosis, Candida krusei, Candida guilliermondii and Candida lusitaniae*) or to the 5S rRNA gene of *S. cerevisiae.*

Conveniently, the probe of this aspect will comprise nucleic acid hybridisable to part or all of the 5S rRNA gene of *C. albicans* strain 10261. The 121 base pair nucleotide sequence for this gene as determined by the applicants is shown in the following nucleotide sequence from nucleotide 937 to nucleotide 1057.

1          11         21         31         41         51
GGATCATACCAAAAAAAAATCGGGGGTAGCGATGAGGTAGTGCAAGTTATACCAGAGAGC
CCTAGTATGGTTTTTTTTTAGCCCCCATCGCTACTCCATCACGTTCAATATGGTCTCTCG

61         71         81         91         101        111
CTCAATTTGAACGTGGTACTGCACACAAACGGTAGCCCTAACCCTAATTAAATGCACGTG
GAGTTAAACTTGCACCATGACGTGTGTTTGCCATCGGGATTGGGATTAATTTACGTGCAC

121        131        141        151        161        171
ACCCACACAATTTTCAACCACCAACAACACACCAAAAATGTATGTACACTCGGAGTGGAA
TGGGTGTGTTAAAAGTTGGTGGTTGTTGTGTGGTTTTTACATACATGTGAGCCTCACCTT

181        191        201        211        221        231
AATATTTCCCCAGGCATAATTGTGGTTGCCAATTATAGTGGAGTGTTTGTTATTAGTATA
TTATAAAGGGGTCCGTATTAACACCAACGGTTAATATCACCTCACAAACAATAATCATAT

241        251        261        271        281        291
GGAGTACTCCTTTTATGTGGAAAAATCGAAAAACATAAAACAGTGCAGCTATACACTAAC
CCTCATGAGGAAAATACACCTTTTTAGCTTTTTGTATTTTGTCACGTCGATATGTGATTG
                                CoARS1

301        311        321        331        341        351
AAATGGTATACCCATGGTCAGACAACACCAAACAACACACCCGTAATGGTTGGTTTGGCT
TTTACCATATGGGTACCAGTCTGTTGTGGTTTGTTGTGTGGGCATTACCAACCAAACCGA
     AccI

361        371        381        391CoARS2 401        411
AAATAGAGCATCTCATTTTGTGTATTATTTTATGTTTTGGATATTTTTAGTTTGAAATTT
TTTATCTCGTAGAGTAAAACACATAATAAAATACAAAACCTATAAAAATCAAACTTTAAA

421        431        441        451        461        471
ATATAAAAATATTTTACTCCAATTTTCTTGCCAAATTTTGTACAAAAAGTAAAAAATAGA
TATATTTTTATAAAATGAGGTTAAAAGAACGGTTTAAAACATGTTTTTCATTTTTTATCT

481        491        501        511        521        531
ACTTCCAATTTTTGTTAAACAAACTTCAATCTAACAACCGGGATAGTGTTTGGGGGGCTG
TGAAGGTTAAAAACAATTTGTTTGAAGTTAGATTGTTGGCCCTATCACAAACCCCCCGAC

541        551        561        571        581        591
TGTAACACCACTGGTGTAATAAAAGTGCCAATATTTGGAAATTAATTTTTTGGTGGTGGA
ACATTGTGGTGACCACATTATTTTCACGGTTATAAACCTTTAATTAAAAAACCACCACCT

601        611        621        631        641        651
CGTTTCTTCCTAAAACACCTAGATGTGCTAGTATAATTGCACAACCAGCACGCACACTGC
GCAAAGAAGGATTTTGTGGATCTACACGATCATATTAACGTGTTGGTCGTGCGTGTGACG

661        671        681        691        701        711
GTCTGGCCGGTCCTGGACTACATTTTGTCTCTAACATTCGTAGCTGCAGCACTGCTACAA
CAGACCGGCCAGGACCTGATGTAAAACAGAGATTGTAAGCATCGACGTCGTGACGATGTT
                                            PstI

721        731        741        751        761        771
CCCATCAATGGCCACCCGGACATACATATTCCCGTTTTGTGCCACAGCCACCCAACTTTC
GGGTAGTTACCGGTGGGCCTGTATGTATAAGGGCAAAACACGGTGTCGGTGGGTTGAAAG

781        791        801        811        821        831
CGGCTCAAAAAATGGCTCCACACAATTTGGGCACCCCGATTACCCCTCCCCTGCAGCGAC
GCCGAGTTTTTTACCGAGGTGTGTTAAACCCGTGGGGCTAATGGGGAGGGGACGTCGCTG
                                                    PstI

841        851        861        871        881        891
AGCACATACACTTTGGTGGTTGTGGGGCTAGAGCCCTAAAAGTGAATTTGGTCACGTGAG
TCGTGTATGTGAAACCACCAACACCCCGATCTCGGGATTTTCACTTAAACCAGTGCACTC

901        911        921        931        941        951
AGACAGCAGATAAGGTTCGCATTTTTTTATTTTTTCGGTTGCGGCCATATCTAGCAGAAA
TCTGTCGTCTATTCCAAGCGTAAAAAAATAAAAAAGCCAACGCCGGTATAGATCGTCTTT

961        971        981        991        1001       1011
GCACCGTTCCCCGTTCGATCAACCGTAGTTAAGCTGCTAAGAGCAATACCGAGTAGTGTA
CGTGGCAAGGGGCAAGCTAGTTGGCATCAATTCGACGATTCTCGTTATGGCTCATCACAT

1021       1031       1041       1051       1061       1071
GTGGGAGACCATACGCGAAACTATTGTGCTGCAATCTATTTTTTTTTAGTAACGTATATT
CACCCTCTGGTATGCGCTTTGATAACACGACGTTAGATAAAAAAAAATCATTGCATATAA

1081       1091       1101       1111       1121       1131
TTTTTTTTGCATGAACCAAGGGTAATCGACCCTGCTCTATCCAGTGATGGTGATAAAGTT
AAAAAAAACGTACTTGGTTCCCATTAGCTGGGACGAGATAGGTCACTACCACTATTTCAA

1141       1151       1161       1171       1181       1191
GGGTGGTGTCTAACAAAAAGGGGGACAACCAGAGGGTGTATAAATAAGGAGAAGCTGGGA
CCCACCACAGATTGTTTTTCCCCCTGTTGGTCTCCCACATATTTATTCCTCTTCGACCCT

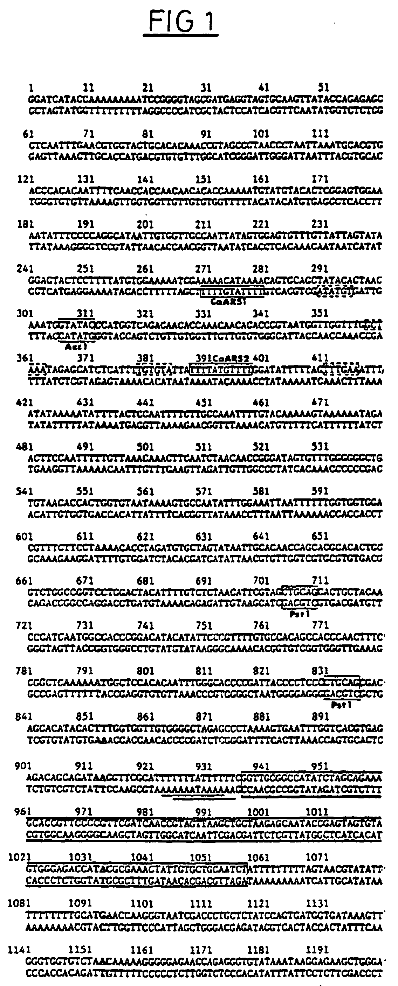

The above nucleotide sequence is referred herein as "SEQ ID No 1", and is also given in Figure 1 of the accompanying drawings.

As will be readily appreciated by the worker having ordinary skill in the art, the probe may take a variety of forms. In this regard, reference can usefully be made to Tenover F C, "Diagnostic Deoxyribonucleic Acid Probes for Infectious Diseases", Clinical Microbiology Reviews 1 82-102 (1988), and Tenover, FC, "DNA Probes for Infectious Diseases", CRC Press Inc, Boca Raton, Florida (1989), which reviewed this area of technology.

As will also be appreciated, the nucleic acid which forms the probe and which is capable of hybridising to DNA and coding the 5S rRNA gene can be DNA or RNA. It is however preferred that the probe be a DNA probe.

The nucleic acid probe can also be capable of hybridising to either the sense or anti-sense strand of DNA coding for the 5S rRNA gene. Conventionally, a probe specific for the sense strand in coding these sequences will be employed.

It will also be appreciated that the probe can vary in terms of size. For example, where the probe is a DNA probe, it can comprise DNA hybridisable to the entire 5S rRNA gene and flanking sequences of the organism's DNA. Indeed, such a probe is preferred. Alternatively, the probe may be in the form of an oligonucleotide probe of from 14 to 40 base pairs in length which is specific for any part of the 5S rRNA gene sequence. Such oligonucleotide probes have the advantage of being stable over time and therefore suitable for use in diagnostic kits. Oligonucleotide probes also hybridise to the target DNA at very rapid rates and are simple to prepare by synthesis using an appropriate synthesizer. An example of such a synthesizer is the Applied Bio-systems DNA synthesizer.

A probe which complies with the applicants' preferences as outlined above which has been found to be particularly suitable for use in the general screening method is a labelled DNA probe in the form of a 0.4 kb *Pst*I/*Bam*H1 digestion fragment derived from *C. albicans* strain 10261. This probe contains a DNA sequence coding for the entire 121 base pair 5S rRNA gene of *C. albicans* strain 10261 and has been found by the applicants to be capable of detecting the presence of any pathogenic fungal organism in a sample as will be more particularly described below.

*C. albicans* strain 10261 is available upon request from the American Type Culture Collection (ATCC) Rockville, Maryland 20852, USA under accession number 10261.

In contrast to the requirements for the probe for use in the general screening assay outlined above, where the method is to be used to specifically identify a particular species of fungal organism as being present in a sample, the preselected region of DNA to which the probe must hybridise is the (or a) non-transcribed spacer region of the rRNA repeat unit. Moreover, in order to be suitable for use in this method for specifically identifying a particular fungal organism, the probe must comprise nucleic acid capable of hybridising to part or all of one strand of a double-stranded DNA molecule containing a non-transcribed spacer region of the rRNA repeat unit of that particular species of organism.

By way of exemplification, where the species of fungal organism to be detected is *C. albicans*, it has been found by the applicants that a probe which hybridises to part or all of the non-transcribed spacer region of the rRNA repeat unit of *C. albicans* strain 10261 can be employed. The nucleotide sequence for this region has been determined by the applicants and is shown in SEQ ID No 1 from nucleotides 1 to 936.

By way of further exemplification, it has additionally been found by the applicants that a probe which hybridises to part or all of the non-transcribed spacer region of the rRNA repeat unit of *S. cerevisiae* can be employed. The nucleotide sequence of this non-transcribed spacer region is as reported by Skryabin *et al.*, Nucleic Acid Research 12 No. 6, 2955-2968 (1984).

It will be appreciated that the non-transcribed spacer region(s) of other species of pathogenic fungi can be similarly isolated from an organism typical of the species. Such isolation can be achieved through the use of PCR amplification of part or all of the non-transcribed spacer region of the selected organism, with the PCR primers being based upon the known sequence of either the non-transcribed spacer region of the rRNA repeat unit or the flanking rRNA coding regions of the organism.

Again, the probe hybridisable to the non-transcribed spacer region can take a variety of forms. However, it is once more preferred that the probe be a DNA probe, that it hybridises to the sense strand of DNA, and that it be labelled.

As with the probe for use in the general screening method, the probe for use in the present method for identifying a particular species of yeast organism can vary in size. In some embodiments, the probe may be hybridisable to the entire non-transcribed spacer region of the organism, while in other embodiments the probe may be in the form of an oligonucleotide or nucleotides hybridisable to any part of the non-transcribed spacer region.

Again, by way of exemplification where the species of organism sought to be detected is *C. albicans,* the applicants have found a labelled probe which comprises DNA hybridisable to only part of the nucleotide sequence of the non-transcribed spacer region set out in SEQ ID No 1 to be suitable for use in the detection method. In particular, a DNA probe which hybridises to nucleotides 1 to 710 of the SEQ ID No 1 sequence has been found to be especially useful in detecting the presence or absence of *C. albicans* species organisms in a sample. This probe is readily obtainable in the form of a 0.9 kb *Pst*I digestion fragment derived by cloning techniques from the genome of *C. albicans* strain 10261.

Once the probe for use in either method outlined above is obtained and optionally labelled, the detection procedure involves two steps. The first of these steps is the testing of a sample with the optionally labelled probe under conditions such that the probe will hybridise to part or all of any DNA present which codes for the target DNA sequence. Accordingly, where such target DNA is present in the sample, a hybrid DNA molecule will result.

The stringency of the hybridisation conditions under which the testing step is performed can be varied as desired through, for example, variations in temperature, salt concentration and formamide concentration. However, it is preferred that the testing step be performed under conditions of low stringency in order to maximise the sensitivity of the assay. In particular, this will allow a probe which is based upon the DNA sequence coding for the 5S rRNA gene of one yeast organism to hybridise to DNA coding for the 5S rRNA gene of any other yeast organism present in the sample, notwithstanding minor variations in the DNA sequence coding for this gene between organisms.

The reaction system which is employed for the testing step may be any of those systems known in the art. However, the system selected will to a great extent depend upon the labelling of the probe. For example, where the probe is not labelled, it can be bound to a solid matrix formed from materials such as nitrocellulose, cellulose or plastic and have sample DNA which has been released by mechanical, chemical, heat sonication or enzymic treatment applied to it. Target DNA complementary to the probe sequence becomes bound to the matrix via the probe. The matrix is washed to remove unbound DNA and then the target DNA is released by heating or transfer to a denaturing solution.

An alternative reaction system involves the use of hydroxyapatite (HAP) which selectively binds double-stranded DNA. Sample DNA obtained as above can be mixed with the probe in single-stranded form in a reaction system designed to allow hybridisation and prevent nuclease action. Any hybrid DNA in the reaction solution is then exposed to and bound by HAP and any unhybridised probe removed by washing or by digestion by single-strand-specific nuclease followed by precipitation with a suitable reagent such as ethanol or trichloroacetic acid.

In contrast, where the probe is labelled, the reaction system employed will depend upon the identity of the label used and will follow the manufacturer's instructions for that labelling system.

Following on from the hybridisation step, the second step involves the detection of the presence or absence of hybrid DNA in the sample. This detection step can again employ any of those detection procedures known in the art. The actual procedure employed will however generally depend upon whether or not the probe was labelled, and if so, upon the labelling system used. For example, where the label is selected from amongst the PhotoGene (Gibco BRL), Chemiprobe (Orgenics) and Enhanced Chemiluminescence (Amersham) labels, the procedure adopted to detect hybrid nucleic acid molecules will vary in accordance with the manufacturers instructions.

While the above method involves the use of nucleic acid probes, a second method for detecting the presence of DNA characteristic of the target organism or organisms involves as a first step amplification of part or all of the characteristic DNA sequence. Again, the identity of the characteristic DNA sequence to be amplified will depend upon whether the method is to be applied as a general screening assay or whether the method is for the detection of a particular fungal organism in the sample.

As before, in the former case, the target DNA sequence selected for amplification will be part or all of the 5S rRNA gene of any one of the target fungal organisms, but preferably part or all of the 5S rRNA gene of *C. albicans* strain 10261, the nucleotide sequence of which is shown in between nucleotides 937 and 1057 of SEQ ID No 1.

Alternatively, in the latter case, the target DNA sequence selected for amplification will be part or all of the sequence containing the non-transcribed spacer region of the rRNA repeat unit of the particular species of fungal organism sought to be detected. By way of example, where the fungal species to be detected is *C. albicans*, the sequence selected for amplification will be based upon SEQ ID No 1 between nucleotides 1 and 936. Alternatively, where the fungal organism to be detected is *S. cerevisiae*, the sequence selected for amplification will be based upon the nucleotide sequence of the non-transcribed spacer region of *S. cerevisiae* given by Skryabin *et al*., Nucleic Acid Research (1984), supra.

It will further be appreciated that while the DNA sequence selected for amplification will often be entirely within the non-transcribed spacer region of the rRNA repeat unit of the particular species of fungal organism, this is not essential. It is also contemplated that the DNA sequence selected for amplification may include both a sequence of nucleotides within the non-transcribed spacer region and a sequence of nucleotides from regions flanking the non-transcribed spacer region (such as the 5S rRNA gene).

Once the DNA sequence to be amplified has been selected the amplification step conveniently involves amplification by polymerase chain reaction (PCR) (Mullis and Fallona, Methods Enzymology, 155 335-350, (1987)). Described generally, PCR amplification involves two oligonucleotide primers that flank the characteristic DNA sequence to be amplified and repeated cycles of heat denaturation of the DNA, annealing of the primers to their complementary sequences, and the extension of the annealed primers with DNA polymerase. These primers hybridise to opposite strands of the target sequence and are oriented so DNA synthesis by the polymerase proceeds across the region between the primers, effectively doubling the amount of the DNA. Moreover, since the extension products are also complementary to and capable of binding primers, each successive cycle essentially doubles the amount of DNA synthesized in the previous cycle. This results in the exponential accumulation of the specific target sequence, approximately 2² where n is the number of cycles.

Where PCR is to be employed and the target DNA sequence is identified, the primers defining the sequence are generated. Suitably, these primers are generated synthetically as described above in relation to the generation of oligonucleotide probes. These oligonucleotide probes can be prelabelled to enable the detection and/or capture of the accumulated product. Examples of suitable labels include biotin used for detection or for capture on an avidin coated solid phase, and fluorescein for detection by fluorescence.

Following amplification by PCR, the presence or absence of the target DNA in amplified quantities is detected. This detection step can involve any suitable procedure with gel electrophoresis, Southern blotting and restriction enzyme digestion being examples. Conveniently, the detection step will employ one of several commercially available detection systems such as AMPLICOR (Roche Molecular Systems, USA) or CAPTAGENE (AMRAD Corporation, Victoria, Australia).

Separate amplification procedures which can be employed are the ligase detection reaction (LDR) and the ligase chain reaction (LCR) (Landegran *et al*., Science 241 1047-1080 (1988)). Described generally, these reactions involve the hybridisation of two oligonucleotide probes to denatured DNA contained in a sample with the probes being selected such that the 3' end of one probe is immediately adjacent the 5' end of the other probe when bound to the target DNA. DNA ligase is then added to covalently link these two oligonucleotides, with the linking only occurring where the entire target DNA sequence complementary to both probes is present.

The hybridisation/ligation procedures can be repeated cyclically using a thermostable DNA ligase to effect linear or exponential amplification of the target DNA depending on whether probes are provided which are hybridisable to one or both strands of the target DNA.

Again, once the amplification is complete, any conventional procedure for detecting the presence or absence of amplified target DNA in the reaction mixture can be employed. It is however preferred that the detection step be effected through the use of differentially labelled oligonucleotide probes, with the presence of the amplified target DNA in the sample being shown through the detection of both labels on a particular DNA molecule.

As yet a further method, the applicants provide an approach to the detection of fungal infection which is a combination of the general and specific assays described above. This combined approach has as its first step the general screening step detailed previously (i.e. testing the sample to detect the presence of part or all of a double-stranded DNA molecule characteristic of and specific for the target organisms, the DNA molecule comprising a nucleotide sequence coding for the 5S rRNA gene of one of the target organisms), and as its second step a species-specific assay which involves testing the sample to detect the presence of part or all of one or more of a group of double-stranded DNA molecules, each DNA molecule being characteristic of and specific for a different target organism and comprising a nucleotide sequence of a non-transcribed spacer of the rRNA repeat unit of that organism.

Conventionally, the second step will provide for the detection of a number of different DNA molecules from within the group at the same time. For example, the sample could conveniently be tested to determine whether or not any DNA coding for the non-transcribed spacer region of the rRNA repeat unit of *C. albicans, C. tropicalis, C. krusei* and *C. glabrata* is present.

In its preferred embodiments, the combination method will employ a DNA amplification procedure, usually PCR, in each testing step. A positive result for the first (general) testing step will be the presence of the selected characteristic DNA molecule in amplified quantities, and will indicate the presence of one or more species of pathogenic fungi in the sample.

A positive result for the second (specific) testing step will be the presence of one or more of the group of different DNA molecules in amplified quantities, and will indicate the species of the fungal organism present in the sample.

The advantage of the combination method will be readily apparent. The first step will reveal the presence of any pathogenic fungal organism in the sample, whereas the second step will reveal whether the fungal organism is one of the species tested for, or is an unknown fungal organism.

As an alternate combined method for the detection of DNA characteristic of the presence of fungal organisms in a sample, described are methods for the testing of the sample with a plurality of labelled nucleic acid probes.

The first sub-step of the testing procedure involves testing the sample with a primary labelled nucleic acid probe hybridisable to part or all of one strand of a double-stranded DNA molecule characteristic of the target yeast organisms. As described previously, the DNA molecule for this probe will hybridise to is the 5S rRNA gene of one of the target organisms, conveniently the 5S rRNA gene of *C. albicans* strain 10261.

As is also described previously, the probe may take a variety of forms and carry any one of a variety of known labels.

The second sub-step of the testing procedure involves testing the sample with at least one secondary labelled nucleic acid probe, with each secondary probe carrying a different label to each other secondary probe as well as to the primary probe. Moreover, each secondary probe must be capable of hybridising to part or all of one strand of a double-stranded DNA molecule characteristic of and specific for a different species of target organism. Again, as described previously, the DNA molecule to which each probe must individually hybridise comprises a nucleotide sequence containing a non-transcribed spacer region of the rRNA repeat unit of the particular target yeast species. For example, where the target species is *C. albicans,* the probe will be hybridisable to the nucleotide sequence of the non-transcribed spacer region of the rRNA repeat unit of *C. albicans* strain 10261 set out between nucleotides 1 and 936 of SEQ ID No 1.

As a further example, where the target species is *S. cerevisiae*, the probe will be hybridisable to the nucleotide sequence of the non-transcribed spacer region of the rRNA repeat unit given by Skryabin *et al*., Nucleic Acid Research (1984), supra.

The secondary labelled probe or probes may once again take a variety of forms and carry any conventional label(s). It must however be borne in mind that each probe must carry a different label to all other probes employed in the method.

While in no way excluding the performance of the present method with a single secondary labelled probe, it will be appreciated that it will be usual for a number of such secondary probes to be used. This will be particularly so where the sample in question is suspected to contain any one or more of a number of different pathogenic fungal species. For example, it is contemplated that the method will commonly employ secondary labelled probes for at least *C. albicans, C. tropicalis, C. krusei* and *C. glabrata.*

It will also be appreciated that the sub-steps of the testing procedure can be performed either sequentially or simultaneously. Furthermore, where the sub-steps are performed sequentially, either sub-step can precede the other.

Once the testing procedure has been completed and the probes (both primary and secondary) hybridised to any DNA characteristic of the target organisms in the sample, the second step of the method involves the detection and identification of the target yeast species present in the sample with reference to the labels carried by the probes. Thus, where the sample contains a single pathogenic yeast species and the sample has been tested with both the primary probe and a secondary probe specific for that yeast species, DNA present in the sample will carry both labels. However, where the sample contains a pathogenic yeast species but has not been tested with a secondary probe specific for that yeast species, DNA present in the sample will carry only a single label, this being the label carried by the primary probe. In this way, as with the amplification procedure described above, the method operates as both a general screening assay and a species-specific yeast identification assay.

While the method can be performed as outlined above, an optional although preferred step involves the addition of a DNA ligase to the sample as part of the LDR or LCR to facilitate the detection of the DNA of a particular fungal species. As described previously, where the primary and secondary probes for a particular fungal species are selected to have adjacent 3' and 5' ends respectively, the ligase will link the probes together, thereby increasing the ease of detection.

The methods and procedures described above for the detection of fungal organisms all focus upon the DNA of the organisms in question. The applicants therefore provide as a still further aspect a method for treating fungal cells to release the fungal DNA they contain so that it can be subjected to the detection procedures outlined previously.

The first step of the DNA releasing method is the centrifugation of an appropriate receptacle (such as an "Isolator" tube) containing a solution of fungal cells and a density gradient centrifugation medium incorporating a cell wall disrupting agent. This centrifugation step is performed in order to separate the fungal cells from at least some of the impurities and/or contaminants present in the original solution through the fact that the fungal cells will move into the centrifugation medium during centrifugation whereas the contaminants, as a rule, will not.

An example of an appropriate centrifugation medium is the modified colloidal silica medium, Percoll (Pharmacia), particularly when at a concentration of between 65% w/v and 80% w/v, more particularly when at a concentration of between 70% w/v and 75% w/v. However, other commercially available centrifugation media such as Ficoll, Ludox and sucrose can also be used.

With regard to the cell wall disrupting agent, any one or more of the commercially available agents can be used. Suitable examples include zymolyase and 2-mercaptoethanol, whether alone or in combination.

The solution of fungal cells to be centrifuged can take a number of forms. One such form is where the fungal cells are contained within a sample of whole blood extracted from a patient. An anticoagulant (such as EDTA) is desirably also included in the solution, optionally together with a blood lysing agent.

In an alternative form, the solution of fungal cells is produced as follows. First, a mixture of an anti-coagulant and whole blood containing fungal cells is centrifuged to provide a particulate phase and a supernatant, with the particulate phase containing the fungal cells. Second, the particulate phase and the supernatant are separated (usually by removal of the supernatant). Finally, an amount of either sterile water or an appropriate buffer is added to form the desired solution.

Following on from the first step outlined above, the next step of the method involves the separation of the centrifugation medium (including the fungal cells) from the supernatant. This step will normally involve the supernatant being removed (e.g. by using a disposable pipette) and discarded, but can also be achieved through recovery of the centrifugation medium.

Once separated from the supernatant, the next step of the method is the incubation of the centrifugation medium for a period of time and at a temperature sufficient for the cell wall disrupting agent to disrupt or dissolve the cell wall of the fungal cells to leave the fungal cells as protoplasts. The time and temperature perameters of the incubation will of course depend upon the cell wall disrupting agent concerned, as well as on the particular fungal species. By way of illustration, where the fungal organism is *C. albicans* and the cell wall disrupting agent is a mixture of zymolyase and 2-mercaptoethanol, the incubation step will continue for about two hours at about 37°C.

The fourth step of the method involves the admixture of the incubated centrifugation medium containing fungal cell protoplasts and an amount of a chelating resin. This resin which is suitably a commercially available chelating resin such as Chelex -100 (BioRad) or Amberlite (Sigma) is added to chelate any metal ions present as blood-sourced contaminants in the centrifugation medium which might otherwise damage or complex with the DNA contained within the fungal protoplasts.

The final step is the heating of the admixture formed as above. This heating step will continue for a period of time and at a temperature sufficient to lyse the fungal protoplasts and to release the fungal DNA in a denatured form. Typically, this will mean heating the admixture to a temperature of at least 96°C for a period of at least 5 minutes with a temperature of about 100°C for about 8 minutes being presently preferred.

The fungal DNA so released is in a form suitable for direct detection using nucleic acid probes, or for amplification by an appropriate amplification procedure, without any further purification being necessary.

The method may also include the recovery of the released fungal DNA if the DNA is not to be immediately subjected to the detection procedures described previously.

While the above steps are those which are essential in the DNA extraction method, the applicants have also found it useful in some instances to perform a number of additional steps. Examples of these additional optional steps include the vortexing or centrifugation of the admixture incorporating the chelating resin both prior and subsequent to the heating step, and the addition of a specific fungal protoplast-lysing agent to the admixture prior to the heating step to assist in the release of the fungal DNA.

The invention will now be illustrated by the following non-limiting examples.

### EXAMPLE 1: PREPARATION OF PROBES

### A: Isolation and purification of 0.9 kb PstI fragment of the non-transcribed spacer region of the C. albicans rDNA repeat unit

The 0.9 kb *Pst*I fragment of the non-transcribed spacer region of the *C. albicans* rDNA repeat unit was selected for use as a probe for the purpose of these Examples. The fragment in question was generated and purified as follows.

DNA derived by cloning techniques from *C. albicans* 10261 was digested with 6 units of *Pst*I per µg of DNA at 37°C for 3 h. Agarose gels for separation of the digested DNA were prepared by the addition of 0.8 g of agarose (BioRad electrophoresis quality) to 2.0 ml 50 x Tris-acetate electrophoresis (TAE) buffer and 98 ml of deionised water. This mixture was melted, cooled to 50 °C and poured into a horizontal gel electrophoresis apparatus (BioRad) (Maniatis *et al.,* Molecular Cloning: A Laboratory Manual, Cold Spring Harbor (1982)). A gel comb was inserted to produce wells and the gel left to set. Once set, the gel was placed in the electrophoresis tank. The tank was filled with gel running buffer (1 x TAE buffer).

Loading dye was added at 1 µl per 10 µl of digested samples for visualisation before loading of the samples onto the gel. The gel of digested DNA was run for 2-3 h at 70-90 V (BioRad Electrophoresis Power Unit) which allowed good separation of the DNA fragments. The gel was then stained for 6 minutes in ethidium bromide (1 µg ml⁻¹), destained in deionised water for 6 minutes, and the DNA bands visualised on a UV light trans-illuminator. Hind III digested lambda phage DNA was run as a control and standard lane on the gel, to enable determination of the relative molecular weights of bands on the gel.

The 0.9 kb DNA band determined from the relative molecular weight of digested lambda DNA was excised from the gel by the following method. A razor cut was made in the gel parallel to, and immediately adjacent to, the 0.9 kb band. A small piece of DEAE paper slightly larger than the band was inserted into the razor cut. The gel was replaced in the electrophoresis apparatus with only sufficient running buffer to allow current to run through the gel, i.e. so that the gel was just immersed but the buffer did not cover the gel. The gel was run at the same voltage as during band separation for a further 10 minutes. The DNA band was by this means bound to the DEAE paper and this was confirmed by use of the transilluminator. The DNA was then eluted by incubation at 37°C for 30 minutes with TE buffer (10 mM Tris-HCl, 0.1 mM EDTA, pH 8.0) containing 1 m NaCl, precipitated with ethanol and resuspended in deionised water or TE buffer at concentration of 1.0 µg ml-1 of DNA. Confirmation of fragment integrity and concentration was carried out by agarose gel electrophoresis.

### B: Isolation and purification of 0.4 kb PstI/BamH1 fragment of the 5S rDNA of C. albicans

The 0.4 kb *Pst*I/*Bam*H1 fragment of the 5S rDNA of *C. albicans* was also selected for use as a probe for the purpose of these Examples. The fragment in question was generated and purified as described above for the 0.9 kb *Pst*I fragment of the non-transcribed spacer region of the *C. albicans* rDNA repeat unit.

### C: Labelling of the fragments

The purified fragments were then labelled with ³⁵SdATP by nick translation (Rigby P W J *et al*.., J Mol Biol 123 387-404 (1977)). The labelled fragments were named probes EOB1 and EOB2, respectively.

### EXAMPLE 2: DETERMINATION OF PROBE SPECIFICITY

### A: Determination of specificity of C. albicans detection by the constructed probes was conducted with the following yeast species and strains:

| Yeast | Strain | Source |
|---|---|---|
| *C. albicans* | 10261 | ATCC |
| | 3153 | NCPF |
| | A72 | Laboratory strain |
| | MEN | Laboratory strain |
| | 473 | Laboratory strain |
| | 85/031 | Laboratory strain |
| | SGY-243 | R Kelly (Merck & Co Inc, Rahway N J) |
| | Ci 001-007 | Dunedin Public Hospital and |
| | 009-013 | School of Dentistry, Otago |
| | 015-041 | University |
| *C. tropicalis* | MY 820567 | NZCDC* |
| | MY 820738 | NZCDC |
| | 13803 | ATCC |
| | Ci 008, 014, 040 | Dunedin Public Hospital and School of Dentistry, Otago |
| University | | |
| *C. krusei* | 89.102 | NZCDC |
| | 90.147 | NZCDC |
| | 89.021 | NZCDC |
| | 89.221 | NZCDC |
| *C. parapsilosis* | 90.493 | NZCDC |
| | 90.454 | NZCDC |
| | 90.111 | NZCDC |
| | 90.463 | NZCDC |
| *C. kefyr* | 78.1161 | NZCDC |
| | 82.656 | NZCDC |
| | Colindale | NZCDC |
| | 87.256 | NZCDC |
| *C. guilliermondii* | 85.791 | NZCDC |
| | 85.739 | NZCDC |
| | 86.117 | NZCDC |
| | 89.140 | NZCDC |

| | | |
|---|---|---|
| *NZCDC - New Zealand Communicable Disease Centre | | |

The strains tested include a total of 50 clinical isolates of *Candida* species. These were collected by the Dunedin Public Hospital between January 1991 and March 1991. All clinical isolates were identified by the Microring YT system (Medical Wire and Equipment Co, UK) and all *C. albicans* strains formed germ-tubes when yeast cells were incubated in serum. The identities of all strains used were confirmed by the use of an immune agglutination identification system, IATRON® (Iatron Laboratories, Tokyo, Japan) which was also used to determine the serotype of *C. albicans* strains. *C. albicans* laboratory strains MEN and A72 and nine of the clinical isolates were serotype B. All other *C. albicans* strains were serotype A. Yeast strains were propagated on YPD agar (containing, per litre, 3.0 g yeast extract, 10.0 g bactopeptone, 20.0 g glucose and 15.0 g agar) and stocks were maintained in YPD containing 15% glycerol at -80°C.

Specificities of the probes were determined by use of a dot blot protocol, using Hybond-N+ membrane Amersham UK, performed as follows. Yeasts were grown at 30°C on YPD agar for 18 h and cells from the surface of the plate were suspended in sterile deionised water at a concentration of 1 x 10⁸ cells ml⁻¹. In a microcentrifuge tube, a portion (20 µl) of this suspension was mixed with 1 M sorbitol containing 10 mM EDTA (0.2 ml). Zymolyase-100T (10 µl, 2 mg/ml) (Seikagaku Kogyo, Tokyo, Japan) was added and the suspension was incubated for 2 h at 37°C to produce spheroplasts. These were lysed by the addition of 20% w/v N-lauroyl sarcosinate (12 µl pH 8.0) in the presence of proteinase K (25 µl, 10 mg/ml) and EDTA (50 µl, 0.4 M, pH 8.0) and incubated for 2 h at 50°C. Portions (0.1 ml) of the DNA extracts were added to the wells of a dot blot manifold containing Hybond N+ membrane (Amersham) and incubated with 0.1 ml 1 M NaOH for 20 min before applying gentle vacuum (∼5 kPa). Blots were washed in ammonium acetate (1 M, pH 5.5) and in 2 x SSC (1 x SSC: 0.15 M NaCl, 15 mM trisodium citrate, pH 7.0), before being used in hybridisation experiments.

Having achieved fixation of the DNA, the membranes were ready for hybridisation to the ³⁵S labelled probes EOB1 and EOB2 prepared in Example 1. Hybridisation conditions were as follows. The hybridization solution contained x5 Denhardt's solution (50 x Denhardt's: 1% w/v BSA, 1% w/v Ficoll, 1% w/v polyvinylpyrollidone in 6 x SSC), 6 x SSC, and 0.5% w/v sodium dodecyl sulphate (SDS). Blots were prehybridised for 3 h at 68°C and then incubated with radiolabelled probe (100 ng, specific activity 1 x 10⁸ dpm/µg) (denatured by boiling in 0.2 M NaOH for 2 min) for 16 h at 68°C in the presence of herring sperm DNA (0.1 mg/ml). High stringency washes were carried out for 2 h at 65°C in 0.1 x SSC containing 0.5% SDS (allowing <10% mismatch), and low stringency washes were performed for 2 h at 50°C in 6 x SSC containing 0.5% SDS (allowing approximately 30% mismatch).

Positive hybridisation on the membranes was detected by exposure of the membranes to radiation-sensitive film (Amersham βmax).

The results of the above testing are given in Table 1.

**TABLE 1:**

| HYBRIDISATION OF DNA FROM *CANDIDA* SPP. AND CLINICAL ISOLATES WITH ³⁵S LABELLED DNA PROBES | | | |
|---|---|---|---|
| YEAST | NUMBER OF STRAINS TESTED | NUMBER OF STRAINS POSITIVE WITH DNA PROBE | |
| | | EOB1 | EOB2 |
| Laboratory strains | | | |
| *C. albicans* | 7 | 7 | 7 |
| *C. tropicalis* | 3 | 0 | 3 |
| *C. parapsilosis* | 4 | 0 | 4 |
| *C. kefyr* | 4 | 0 | 4 |
| *C. krusei* | 4 | 0 | 4 |
| *C. guilliermondii* | 4 | 0 | 4 |
| | | | |
| Clinical isolates | | | |
| *C. albicans* | 38 | 38 | 38 |
| *C. tropicalis* | 4 | 0 | 4 |
| Typical examples of these results are shown in Figure 3. | | | |

B: By way of further illustration of probe specificity, hybridisation of the probes to known concentrations of purified DNA from a number of sources was examined. RNA-free genomic DNA was prepared from *C. albicans* ATCC 10261 and *C. tropicalis* MY 820738 by the method of Cryer *et al.,* Methods Cell Biol 12 39-44 (1975). Human DNA was purified from peripheral blood lymphocytes as described by Hermann and Frischauf (Methods in Enzymology, Berger S L and Kimmel A R, Eds, Academic Press. Vol. 152, 180-182). *Streptococcus sanguis* DNA was prepared by the method of Jenkinson, J Gen Microbiol 133 1909-1918 (1987) and *Listeria moncytogenes* DNA was prepared by the method described by Flamm *et al.,* Infect Immunol 44 157-161 (1984). DNA concentrations of purified preparations were measured by absorbance at 260 nm.

Neither probe EOB1 or EOB2 prepared as in Example 1 hybridised to dot blots of human lymphocyte DNA nor to DNAs from two bacteria, *Streptococcus sanguis* and *Listeria monocytogenes.* Probe EOB1 did not hybridise with DNAS other than from *C. albicans* when reacted with high concentrations of purified DNA (0.2 µg yeast, bacterial or human DNA per dot blot), whereas hybridisation to *C. albicans* genomic DNA was clearly detectable with dot blots of <200 pg purified DNA.

From the above results, probe EOB1 was shown to detect specifically *C. albicans* DNA (Table 1 and Fig. 1). The specificity was 100% for 45 known strains of *C. albicans* compared with 23 strains of five other *Candida* species.

The absence of hybridisation of this probe to genomic DNA from other yeast species was not due to inefficient extraction of DNA from the other yeasts. This was shown firstly because duplicate blots of yeast genomic DNAs did hybridise with the second non-specific probe EOB2, and secondly because known concentrations of purified DNA from yeasts and also from human and bacterial cells did not hybridise.

Probe EOB2 was shown to hybridise to all strains of six species of *Candida* listed in Table 1, but not to human or bacterial DNA under conditions of high stringency.

### EXAMPLE 3: DETERMINATION OF PROBE SENSITIVITY

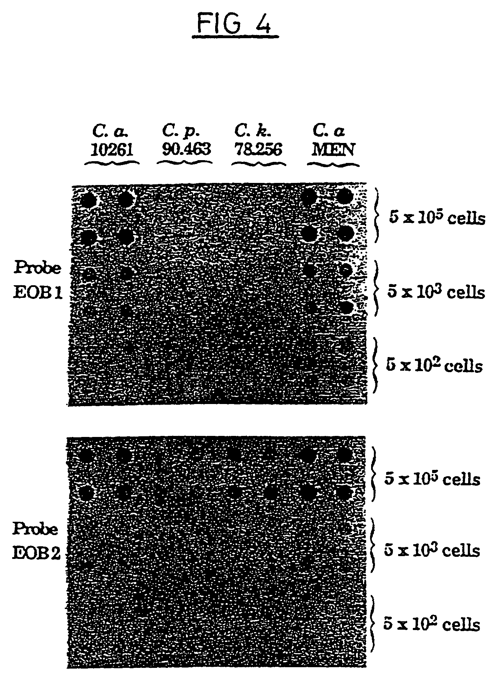

Serial dilutions of two strains of *C. albicans* and two other *Candida* species were used to determine the sensitivities of the constructed probes EOB1 and EOB2. Yeast cell suspensions were serially diluted in sterile deionised water and DNA was extracted from the cells present in 20 µl samples. DNA extraction, immobilisation on nylon membrane and hybridisations were carried out at high stringency as described in Example 2. As shown in Figure 4, probe EOB1 gave a positive hybridisation signal, under high stringency conditions, with DNA extracted from a diluted cell sample containing 5 x 10² cells (but not from a 10 fold lower dilution) for the two *C. albicans* strains tested. At the high stringency used, probe EOB2 was less sensitive for *C. albicans* DNA; the detection limit observed was 5 x 10³ cells (Fig. 4) and a detection limit of 5 x 10⁵ cells was observed for two other *Candida* species (*C. parapsilosis* and *C. krusei*).

### EXAMPLE 4: DETECTION OF C. ALBICANS IN SAMPLES OF HUMAN BLOOD

### A. BY DNA PROBE

The usefulness of a DNA probe as a diagnostic tool is determined by the ability to recover DNA from low numbers of the target organism in clinical specimens as well as by probe specificity and sensitivity. The probe EOB1 was used to investigate the recovery of *C. albicans* cells and DNA from whole human blood inoculated with yeast cells.

A method was developed, similar in principle to the lysis centrifugation method (Dorn G L, Smith, K, J Clin Microbiol 7 52-54 (1978)) that allowed yeast cells to be recovered and concentrated, with minimal contaminating blood material. It was observed that when centrifuged, *C. albicans* cells sedimented through Percoll® at concentrations ≤80% (v/v in PBS) whereas blood cells floated on concentrations ≥65% (v/v). Therefore a 75% solution of Percoll® was used as a cushion during centrifugation of blood inoculated with *C. albicans* cells. The yeast cells were pelleted whereas the majority of the blood material could be removed with the upper layer. After diluting the Percoll®, and recentrifugation, the yeast cells could be counted or subjected to DNA extraction procedures.

The method used was as follows. Whole blood from human volunteers was collected into Vacutainer® tubes containing EDTA as anticoagulant and yeast cells of *C. albicans* MEN were added at known concentrations (final concentrations between 5 x 10⁵ and 50 cells/ml). Blood (1.0 ml) containing yeast cells was layered onto 0.3 ml of 70% Percoll® (Pharmacia) and centrifuged at 7,500 g for 10 min at room temperature. The top layer containing blood material was removed and the Percoll® containing the yeast cell pellet was diluted (1:1 v/v) with distilled water and the pellet washed once by centrifugation before suspension in sterile distilled water. The pellet was suspended in water to determine viable cell numbers (see below), or DNA was extracted as follows:

Zymolyase-100T (10µl, 2 mg/ml) (Seikagaku Kogyo, Tokyo, Japan) was added and the suspension was incubated for 2 h at 37°C to produce spheroplasts. These were lysed by the addition of 20% w/v N-lauroyl sarcosinate (12 µl pH 8.0) in the presence of proteinase K (25 µl, 10 mg/ml) and EDTA (50 µl, 0.4 M, pH 8.0) and incubated for 2 h at 50°C.

The proportion of the initial inoculum recovered using this method was determined by viable plate counts or by total cell counts using a haemocytometer. To measure viable counts, 0.1 ml appropriately diluted portions of inoculum, or of recovered pellet, were spread on YPD plates which were incubated for 16 h at 30°C. The recovery of yeast cells (as determined by viable count or microscopic observation) using the above method was between 70% and 80%.

Using the probe EOB1 it was possible to detect DNA from 5 x 10² cells in 1.0 ml of blood. Portions (0.1 ml) of the DNA extracts were added to the wells of a dot blot manifold containing Hybond N+ membrane (Amersham) and incubated with 0.1 ml 1 M NaOH for 20 min before applying gentle vacuum (-5 kPa). Blots were washed in ammonium acetate (1 M, pH 5.5) and in 2 x SSC (1 x SSC: 0.15 M MaCl, 15 mM trisodium citrate, pH 7.0), before being used in hybridization experiments.

Having achieved fixation of the DNA, the membranes were ready for hybridization to the 35S labelled probes prepared in Example 1. Hybridization conditions were as follows. The hybridization solution contained x5 Denhardts solution (1% w/v BSA, 1% w/v Ficoll, 1% w/v polyvinylpyrollidone in 6 x SSC), 6 x SSC, and 0.5% w/v sodium dodecyl sulphate (SDS). Blots were prehybridized for 3 h at 68°C and then incubated with radiolabelled probe (100 ng, specific activity 1 x 10⁸ dpm/µg) (denatured by boiling in 0.2 M NaOH for 2 min) for 16 h at 68°C in the presence of herring sperm DNA (0.1 mg/ml). High stringency washes were carried out for 2 h at 65°C in 0.1 x SSC containing 0.5% SDS (allowing <10% mismatch), and low stringency washes were performed for 2 h at 50°C in 6 x SSC containing 0.5% SDS (allowing approximately 30% mismatch).

Positive hybridization on the membranes was detected by exposure of the membranes with radiation-sensitive film (Amersham BMax).

From the above results, probe EOB1 was shown to be specific for *C. albicans.* These results show that this probe can be used for the detection of low concentrations of *C. albicans* cells (5 x 10² in 1.0 ml) in human blood samples.

### B. BY PCR

An experiment similar to that described in Example 4A was performed utilising the "Isolator" blood tube system and PCR rather than hybridization for detection of extracted DNA. 10 ml of human blood was drawn and added to an "Isolator" blood tube. The tube was inverted to mix the contents, *C. albicans* yeast cells were added as described in Example 4A and the tube centrifuged to sediment particulate matter including the added yeast cells. The supernatant was removed with a plastic pipette as prescribed by the protocol for the "Isolator" tube and discarded and 10 ml of sterile distilled water added to the tube. The tube was centrifuged again and the supernatant removed and discarded. The pellet and a small amount of accompanying supernatant was transferred to another tube which contained 0.5 ml of Percoll, 75% in saline. The Percoll cushion also contained Zymolyase (25 µg) and B-Mercaptoethanol (5.0 µl). The tube was centrifuged to pellet the yeast cells into the cushion material. The top layer was removed and the cushion material containing the yeast cells was incubated at 37°C for 2 h to produce protoplasts. To a 50 µl sample, 10 µl of PCR buffer (10 x concentrate, Amersham) was added to lyse the cells. The preparation was then boiled for 8 min with 50 µl of a 50% suspension of the chelating resin Chelex. After centrifuging to pellet the Chelex, samples were taken for PCR amplification. Successful amplification was obtained with a sample containing only 15 *C. albicans* cells.

### EXAMPLE 5: PCR AMPLIFICATION OF TARGET DNA FROM FUNGAL GENOMIC DNA

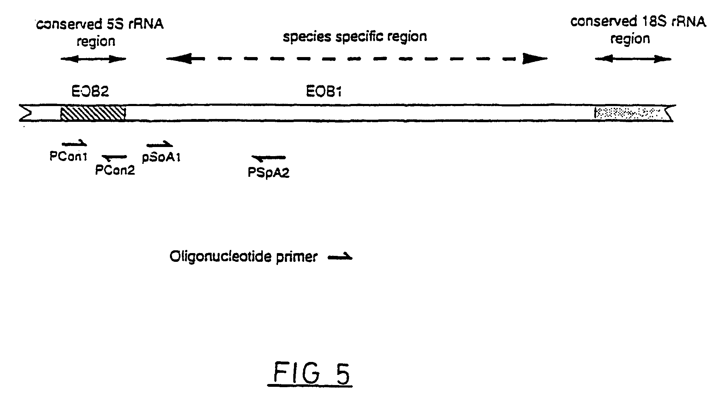

Amplification techniques such as PCR provide a means of increasing the sensitivity of detection systems. To demonstrate its application in the present context, selected strains were used to show the specificity and sensitivity of PCR amplification using primers homologous with either, part of the non-transcribed spacer region (within the EOB1 region: nucleotides 1-936 of SEQ ID No 1) or, part of the 5S RNA coding sequence (within the EOB2 region: nucleotides, 937-1057, SEQ ID No 1). The primer sequences selected in the first case were designated EOB1 primers PSpA1 and PSpA2 and had the sequences 5'TAG CGA TGA GGT AGT GCA AGT3' (SEQ ID No 2) and 5'GCT GCA GCT ACG AAT GTT AG3' (SEQ ID No 3). The primer sequences selected in the second case were designated EOB2 primers PCon1 and PCon2 and had the sequences 5'AGT TTC GCG TAT GGT CTC CC3' (SEQ ID No 4) and 5'GTT GCG GCC ATA TCT AGC AG3' (SEQ ID No 5) (see Figure 5).

Genomic DNA extracted from *C. albicans* laboratory strains 10261, A72, 3153 and randomly selected clinical isolates (Ci 002, 011, 012, 036, 060, 061) (sources as shown in the table in Example 2 above and prepared as described in Example 2) was diluted 100-fold and 10 µl samples used as template in reaction mixes containing dNTPs (25 µM); PCR buffer (Amersham) 10µl; either EOB1 primers PSpA1 and PSpA2, or EOB2 primers PCon1 and PCon2; Taq polymerase, 1 unit; water to 100 µl. Samples were subjected to temperature cycling in a heat block (Hybaid) as follows: 95°C, 25 sec; 55°C, 40sec; 72°C, 50 sec., for 32 cycles.

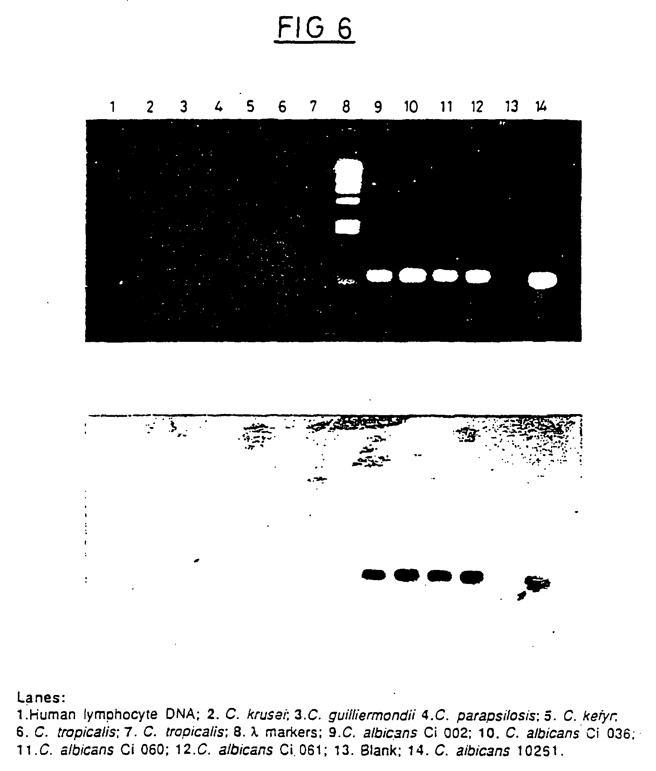

A representative agarose gel and Southern blot showing amplification products using the EOB1 primers PSpA1 and PSpA2 are shown in Figure 6. Amplification products using target DNA from *C. albicans* strains were the predicted length (700 bp); none were detectable using target DNA from five other yeasts and human DNA. The Southern blot was probed with an ECL (Amersham) labelled *Alu*1 fragment of EOB1 (situated internally between the PSpA1 and PSpA2 primer sites). Products from *C. albicans* template DNA hybridised with the *Alu*1 probe, whereas no hybridizing DNA was amplified from five other yeasts and human DNA.

When EOB2 primers PCon1 and PCon2 were used in amplifications, template from all *C. albicans* strains and from five other yeast species DNAs but not from human genomic DNA gave product that hybridized with a *C. albicans* EOB2 probe. In each case the amplified product was of the predicted size (105 bp).

The DNA extraction procedure was modified in experiments to determine the sensitivity of detection by PCR, because if undiluted, some components of the extraction mix were found to inhibit the amplification reaction. In these experiments, EDTA was omitted and protoplasts were lysed by the addition of PCR buffer, not Sarcosyl. After heating at 95°C for 10 min to destroy proteinase activity; 60 µl samples were used directly in the amplification procedure. Yeast cells of *C. albicans* were serially diluted and duplicate samples taken for enumeration by viable counting and for DNA extraction and amplification using the EOB1 primers and conditions as described above. Product was detectable by agarose gel electrophoresis when the DNA from 10-20 cells (determined by viable count) was used as template.

### EXAMPLE 6: AMPLIFICATION IN A SINGLE PCR OF ADJACENT DNA SEQUENCES IN MULTIPLEX AND "SEMI-NESTED" PROCEDURE

The juxtaposition of conserved (EOB2) and unique (EOB1) sequences in the rRNA DNA repeat region (Figure 5) enables development of multiplex and nested PCR to incorporate both specificity and selectivity in a single amplification. When the three primers, PCon1, PCon2 (from the EOB2 sequence) and PSpA2 (from the EOB1 sequence) were used in a "semi-nested" PCR amplification under conditions as described in Example 5, two products were obtained with *C. albicans* 10261 template DNA (prepared as described in Example 5). However, only the EOB2 (conserved) product was obtained with template DNA from other yeast DNAs.

Similarly, in amplifications using PCon1 and PCon2 in addition to a primer, PSpB, from the equivalent region to EOB1 in *S. cerevisiae,* (sequence 5'GGT GAC GGA AAT ACG CTT CA3', SEQ ID No 6) two products were detected with *S. cerevisiae* DNA but only the EOB2 product was detected with *C. albicans* DNA. Furthermore, the experiment showed that multiplex PCR could be used with primers from the rRNA repeat region; when both *C. albicans* and *S. cerevisiae* template DNAs were present in a multiplex PCR together with the primers PCon1, PSpA2 and PSpB, then two products of the predicted sizes derived from both template DNAs were detected.

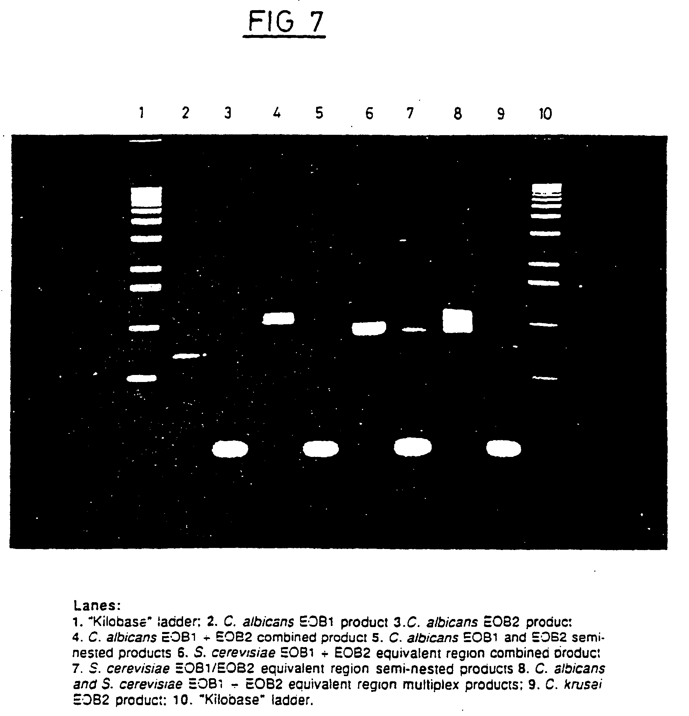

Figure 7 shows a representative agarose gel of the PCR products obtained in the reactions described. The amplification products were detected both by gel electrophesis, and by using the CAPTAGENE (AMRAD Corporation, Victoria, Australia) detection system.

### EXAMPLE 7: SPECIFIC PCR AMPLIFICATION OF PART OF THE NON-TRANSCRIBED RNA SPACER REGION OF S. CEREVISIAE.

A portion of the sequence of the non-transcribed spacer region of the rRNA repeat unit of the yeast *S. cerevisiae* (Skryabin *et al.,* Nucleic Acids Research (1984), supra) was found to be specifically amplified using primers with sequences from within this region. A PCR amplification assay was employed, using the same conditions as described in Example 5, but containing the following primers: PCon1, the sequence of which is homologous with the conserved 5S rRNA coding region (see Example 5) and primer PSpB (see Example 6), which was homologous with 20 nucleotides of the *S. cerevisiae* non-transcribed spacer region 793 nucleotides distant from the 5S rRNA coding region. When *S. cerevisiae* genomic DNA was used as template DNA in the PCR amplification, product of the predicted size (898 bp) was obtained whereas no product was obtained when *C. albicans* genomic DNA was included as target DNA. Therefore the portion of the non-transcribed spacer region of *S. cerevisiae* homologous to primer PSpB is unique to that species.

In a further aspect, diagnostic kits for use in the detection of pathogenic fungi in a medium are provided . In one embodiment, such kits include as components nucleic acid probes with the identity of the probe being dependent on whether the probe is to be used as part of a general screening assay or as part of an assay for specifically detecting a particular fungal organism. For the former, the probe will comprise nucleic acid capable of hybridising under appropriate conditions to one strand of a DNA duplex coding for the 5S rRNA gene of *C. albicans* strain 10261, whereas for the latter the probe will comprise nucleic acid capable of hybridising under appropriate conditions to one strand of a DNA duplex containing the non-transcribed spacer region of the particular fungal organism sought to be detected.

Optionally, the kit will include probes suitable for use in both a general screening assay and a specific detection assay. Furthermore, in such embodiments, the kit will include probes hybridisable to the non-transcribed spacer region of the rRNA repeat unit of a number of different fungal organisms.

The probe employed in the kit will normally be labelled for convenience of use. Any label available in the art capable of revealing the presence of the probe can be used. By way of illustration, the probe can be labelled with ³⁵S dATP as previously exemplified. Other examples of labelling systems which can be employed are the enhanced chemiluminescence (ECL) system available from Amersham, the PhotoGene system available from Gibco BRL and the Chemiprobe system available from Orgenics Limited.

In addition to the optionally labelled probe, the kit may also include one or more of the following:
(a) a lysing agent capable of lysing the cells in the sample of the medium to be tested;
(b) a denaturing solution;
(c) a neutralising solution;
(d) an alkaline fixation solution; and
(e) Standard Saline Citrate (SSC).

Each of the above components can be selected from those known and available in the art and provided in appropriate amounts.

The kit would normally also include instructions for use. By way of convenient illustration, the diagnostic kit can be provided for detecting fungal organisms in a medium by the centrifugation and dot blotting protocol. In such an embodiment, the instructions would direct that a 10 ml sample of the medium (for example EDTA treated blood) be centrifuged in a tube containing a cushion of high density liquid. The cushion would then be washed once with an equal volume of sterile deionised water before recentrifugation. The supernatant would be removed and the pellet resuspended in sorbitol/EDTA and prepared for hybridisation with the probe using the procedures specified in the Examples.

It will also be appreciated that alternately formatted diagnostic kits will be provided where the detection method employs an amplification procedure such as PCR. Exemplary formats for such kits are as follows:

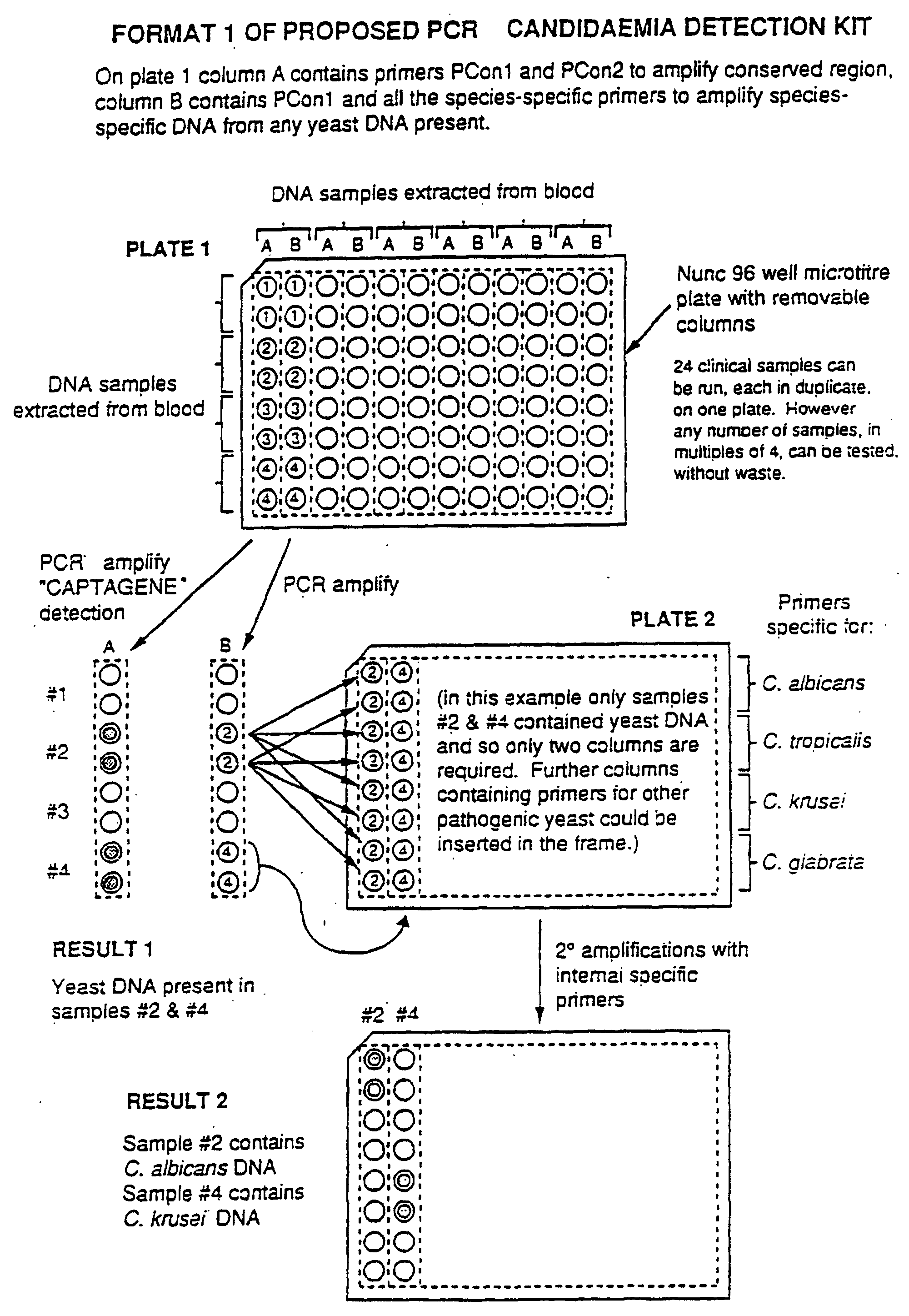
FORMAT 1 OF PROPOSED PCR CANDIDAEMIA DETECTION KIT
On plate 1 column A contains primers PCon1 and PCon2 to amplify conserved region,
column B contains PCon1 and all the species-specific primers to amplify species-
specific DNA from any yeast DNA present.

DNA samples extracted from blood
A B A B A B A B A B A B
PLATE 1
Nunc 96 well microtitre
plate with removable
columns
24 clinical samples can
be run, each in duplicate.
on one plate. However
any number of samples, in
multiples of 4, can be tested.
without waste.
DNA samples
extracted from blood
1 1
1 1
2 2
2 2
3 3
3 3
4 4
4 4

PCR amplify
"CAPTAGENE"
detection
PCR amplify
A
B
#1
#2
#3
#4
2
2
4
4
PLATE 2
Primers
specific for:
2 4
2 4
2 4
2 4
2 4
2 4
2 4
2 4
(in this example only samples
#2 & #4 contained yeast DNA
and so only two columns are
required. Further columns
containing primers for other
pathogenic yeast could be
inserted in the frame.)
C. albicans
C. tropicalis
C. krusei
C. glabrata

RESULT 1
Yeast DNA present in
samples #2 & #4
2° amplifications with
internal specific
primers
#2 #4
RESULT 2
Sample #2 contains
C. albicans DNA
Sample #4 contains
C. krusei DNA

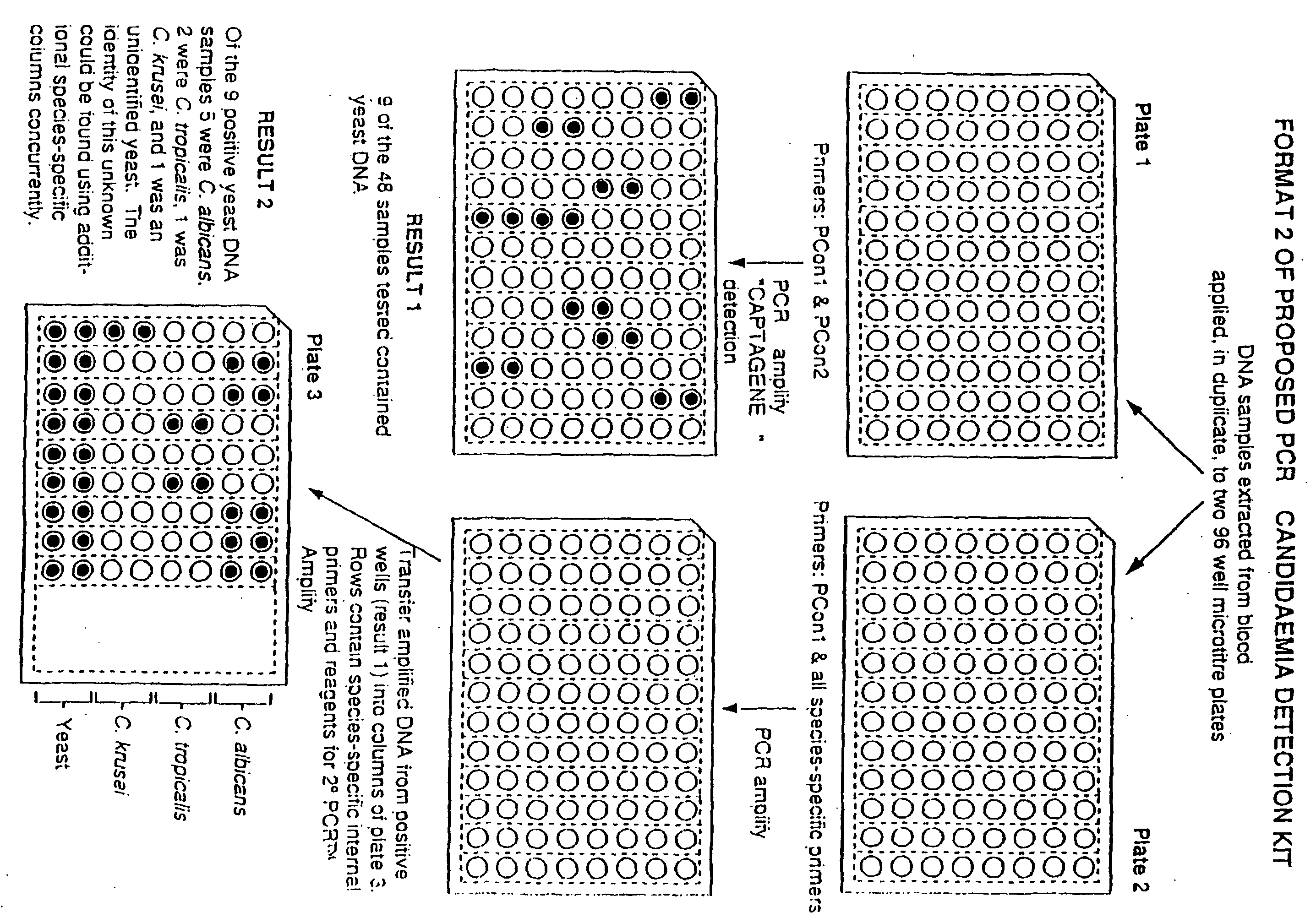
FORMAT 2 OF PROPOSED PCR   CANDIDAEMIA DETECTION KIT

DNA samples extracted from blood
applied, in duplicate, to two 96 well microtitre plates

Plate 1


Plate 2


Primers: PCon1 & PCon2

PCR   amplify
"CAPTAGENE"
detection

Primers: PCon1 & all species-specific primers

PCR amplify


RESULT 1
9 of the 48 samples tested contained
yeast DNA


Transfer amplified DNA from positive
wells (result 1) into columns of plate 3.
Rows contain species-specific internal
primers and reagents for 2° PCR[?]
Amplify

Plate 3


C. albicans
C. tropicalis
C. krusei
Yeast

RESULT 2
Of the 9 positive yeast DNA
samples 5 were C. albicans.
2 were C. tropicalis, 1 was
C. krusei, and 1 was an
unidentified yeast. The
identity of this unknown
could be found using addit-
ional species-specific
columns concurrently.

As will be understood from these formats, such kits will include paired nucleic acid primers which are capable of binding to and which define the characteristic DNA sequence to be amplified, optionally together with other reagents conventionally included in diagnostic kits associated with labelling and detection systems (such as the AMPLICOR and CAPTAGENE systems).

### APPLICATION OF THE INVENTION

Thus, in accordance with the present invention there is provided a method for detecting the presence of fungi in a sample such as a sample of blood or tissue which is both specific and sensitive in terms of being able to detect low numbers of fungal organisms. This is of particular importance for the detection of fungi cells in blood, which is an indicator of disseminated infection, and for which currently available techniques are slow and insensitive. The present method therefore represents a considerable advance over those techniques currently employed, such as antigen/antibody detection methods or viable cell counts, in the detection of candidaemia.

More particularly, in one preferred embodiment described is a system which not only enables the detection of pathogenic fungi in a blood sample by amplification of a conserved repetitive DNA region, but in addition allows identification of the species of fungus present by concurrent amplification of an adjacent species-specific sequence, which can then be identified either by secondary amplification (usually by PCR) or by hybridization techniques.

The system utilises the juxtaposition of conserved and specific sequences in a repeated region of the genome. Targetting these adjacent sequences confers several advantages:
(i) It is a repeated sequence so multiple target sequences are available for amplification;
(ii) A major advantage of adjoining conserved and specific sequences is that the DNA for species identification can be amplified from a single blood sample, with only one additional primer necessary for each species to be identified. Thus the system minimises the number of primers in the PCR, and repeated PCRs on separate blood samples are not required; and
(iii) This system combines a general fungal identification with a species-specific identification in a multiplex semi-nested technique which virtually eliminates the possibility of false positives.

It will be appreciated by those persons skilled in the art that the description provided above is by way of example only and that the present invention is limited only by the following claims.

## Claims

1. A method of detecting the presence of a target fungal organism in a sample comprising the step of testing said sample to detect the presence or absence of part or all of a double-stranded DNA molecule characteristic of and specific for said organism, said DNA molecule comprising a nucleotide sequence containing a non-transcribed spacer region of the rRNA repeat unit of said organism, wherein detecting the presence or absence of said characteristic DNA molecule involves the use of a nucleic acid probe capable of hybridising to a preselected region of the rRNA repeat unit, wherein the target organism is *Candida albicans*, and wherein the nucleic acid probe has a nucleotide sequence comprising part or all of the nucleotide sequence from nucleotide 1 to nucleotide 936 of SEQ. ID No. 1 ; or the nucleic acid probe consists of a 0.9 kb *Pst*I digestion fragment derived from the rRNA repeat unit of *C. albicans* strain 10261 (ATCC 10261), a sub-fragment thereof, or has a nucleotide sequence which hybridises at high stringency thereto.

2. A method according to claim 1 wherein the testing step includes the sub-steps of:
(a) amplifying any DNA present in said sample having a nucleotide sequence of part or all of a non-transcribed spacer region of the rRNA repeat unit of the target organisms;
(b) detecting the presence or absence of said amplified DNA in said sample

3. A method according to claim 2 wherein the amplification procedure employed is the polymerase chain reaction, the ligase detection reaction or the ligase chain reaction.

4. A method according to claim 1 wherein the testing step comprises:
(a) testing said sample with said probe, wherein said probe is optionally labelled and is capable of hybridising to part or all of one strand of said DNA molecule, said testing being performed under conditions such that said probe will hybridise to any DNA containing a non-transcribed spacer region of the rRNA repeat unit of the target organism which is present in the sample to form hybrid DNA;
(b) detecting the presence or absence of said hybrid DNA in the sample.

5. A method according to claim 4 wherein the testing is performed under low stringency hybridisation conditions.

6. A method according to any one of claims 1 to 5 wherein said sample contains DNA extracted from blood or tissue.

7. A method of detecting the presence of one or more of a group of target fungal organisms in a sample comprising the steps of:
(a) testing said sample to detect the presence of part or all of a double-stranded DNA molecule characteristic of and specific for said organism, said DNA molecule comprising a nucleotide sequence coding for the 5S rRNA gene of one of said organisms; and
(b) separately testing said sample to detect the presence of one or more of a group of double-stranded DNA molecules, or part thereof, each said double-stranded DNA molecule being characteristic of and specific for a different target organism and comprising a nucleotide sequence of a non-transcribed spacer region of the rRNA repeat unit of that organism, said testing comprising the use of a method according to any one of claims 1 to 6.

8. A method according to claim 7 wherein testing step (a) comprises the sub-steps of:
(i) amplifying any DNA present in said sample having a nucleotide sequence of the 5S rRNA gene of a target organism; and
(ii) detecting the presence or absence of any amplified DNA in said sample.

9. A method according to claim 7 or claim 8 wherein testing step (b) comprises the sub-steps of:
(i) amplifying any of said group of DNA molecules present in said sample; and
(ii) detecting the presence or absence of any of said group of DNA molecules in amplified quantities.

10. A method according to any one of claims 7 to 9 wherein said sample contains DNA released by the steps of:
(a) centrifuging a receptacle containing a solution of fungal cells and a density gradient centrifugation medium incorporating a cell wall disrupting agent, said centrifugation being performed under conditions such that at least the major proportion of fungal cells move out of solution into said centrifugation medium, resulting in the formation of a supernatant and a centrifugation medium containing fungal cells and said cell wall disrupting agent;
(b) separating said supernatant and said centrifugation medium;
(c) incubating said centrifugation medium for a period of time and at a temperature sufficient for said cell wall disrupting agent to disrupt the cell wall of the fungal cells present in said medium to form fungal cell protoplasts;
(d) admixing said incubated centrifugation medium containing fungal cell protoplasts and an amount of a chelating resin; and
(e) heating said admixture for a period of time and at a temperature sufficient to lyse said protoplasts and to release fungal DNA in a denatured form.

## Patentansprüche

1. Verfahren zur Feststellung der Anwesenheit eines Ziel-Pilzorganismus in einer Probe, das den Schritt der Prüfung der Probe zur Feststellung der Anwesenheit oder des Fehlens eines Teils oder des gesamten doppelsträngigen DNA-Moleküls umfaßt, das den genannten Organismus charakterisiert und für diesen spezifisch ist, wobei das DNA-Molekül eine Nucleotidsequenz aufweist, die einen nicht-transkribierten Spacer-Abschnitt der rRNA-Repeateinheit des genannten Organismus enthält, wobei die Feststellung der Anwesenheit oder des Fehlens des charakteristischen DNA-Moleküls die Verwendung einer Nucleinsäuresonde einschließt, die in der Lage ist, mit einem ausgewählten Bereich der rRNA-Repeateinheit zu hybridisieren,
wobei der Zielorganismus Candida albicans ist,
und wobei die Nucleinsäuresonde eine Nucleotidsequenz aufweist, die einen Teil oder die Gesamtheit der Nucleotidsequenz von Nucleotid 1 bis Nucleotid 936 von SEQ ID Nr. 1 aufweist; oder
die Nucleinsäuresonde aus einem 0,9 kb Fragment eines PstI-Verdaus besteht, das sich ableitet von der rRNA-Repeateinheit des C. albicans-Stamms 10261 (ATCC 10261), einem Unterfragment davon, oder eine Nucleinsäuresequenz aufweist, die mit hoher Stringenz damit hybridisiert.

2. Verfahren nach Anspruch 1, wobei der Prüfschritt die Teilschritte umfaßt:
(a) Amplifizieren der gesamten DNA, die in der Probe vorhanden ist und eine Nucleotidsequenz eines Teils oder der Gesamtheit eines nicht-transkribierten Spacer-Abschnitts der rRNA-Repeateinheit des Zielorganismus aufweist;
(b) Feststellen der Anwesenheit oder des Fehlens der amplifizierten DNA in der Probe.

3. Verfahren nach Anspruch 2, bei dem die angewandte Amplifizierungsprozedur die Polymerase-Kettenreaktion, die Ligase-Nachweisreaktion oder die Ligase-Kettenreaktion ist.

4. Verfahren nach Anspruch 1, bei dem die Prüfstufe umfaßt:
(a) Prüfen der Probe mit der genannten Sonde, wobei die Sonde ggf. markiert und in der Lage ist, mit einem Teil oder der Gesamtheit eines Strangs des genannten DMA-Moleküls zu hybridisieren, wobei die Prüfung unter solchen Bedingungen durchgeführt wird, daß die Sonde mit der gesamten DNA hybridisiert, die einen nicht-transkribierten Spacer-Abschnitt der rRNA-Repeateinbeit des Zielorganismus enthält, der in der Probe vorhanden ist, um eine hybride DNA zu bilden;
(b) Feststellenen der Anwesenheit oder des Fehlens der hybriden DNA in der Probe.

5. Verfahren nach Anspruch 4, bei dem die Prüfung unter Hybridisierungsbedingungen niedriger Stringenz durchgeführt wird.

6. Verfahren nach irgendeinem der Ansprüche 1 bis 5, bei dem die Probe eine aus Blut oder Gewebe extrahierte DNA enthält.

7. Verfahren zur Feststellung der Anwesenheit von einem oder mehreren Ziel-Pilzorganismen aus einer Gruppe von Organismen in einer Probe, das die Schritte umfaßt:
(a) Prüfen der Probe zur Feststellung der Anwesenheit eines Teils oder des gesamten doppelsträngigen DNA-Moleküls, das den genannten Organismus charakterisiert und für diesen spezifisch ist, wobei das DNA-Molekül eine Nucleotidsequenz aufweist, die für das 5S rRNA-Gen von einem der genannten Organismen codiert; und
(b) getrennt davon Überprüfen der Probe zur Feststellung der Anwesenheit von einem oder mehreren aus einer Gruppe von doppelsträngigen DNA-Molekülen, oder eines Teils davon, wobei jedes doppelsträngige DNA-Molekül für einen anderen Zielorganismus charakteristisch und für diesen spezifisch ist und eine Nucleotidsequenz eines nicht-transkribierten Spacer-Abschnitts der rRNA-Repeateinheit des genannten Organismus umfaßt, wobei die Prüfung die Anwendung eines Verfahrens nach irgendeinem der Ansprüche 1 bis 6 umfaßt.

8. Verfahren nach Anspruch 7, bei dem der Prüfschritt (a) die Teilschritte umfaßt:
(i) Amplifizieren der gesamten in der Probe vorhandenen DNA, die eine Nucleotidsequenz des 5S rRNA-Gens eines Zielorganismus aufweist; und
(ii) Feststellen der Anwesenheit oder des Fehlens irgendeiner amplifizierten DNA in der Probe.

9. Verfahren nach Anspruch 7 oder Anspruch 8, bei dem der Prüfschritt (b) die Teilschritte umfaßt:
(i) Amplifizieren von jeglichem DNA-Molekül aus der genannten Gruppe, das in der Probe vorhanden ist; und
(ii) Feststellen der Anwesenheit oder des Fehlens von irgendeinem DNA-Molekül aus der genannten Gruppe in amplifizierten Mengen.

10. Verfahren nach irgendeinem der Ansprüche 7 bis 9, bei dem die Probe eine DNA enthält, die durch die Schritte freigesetzt wurde:
(a) Zentrifugieren eines Gefäßes, das eine Lösung von Pilzzellen und ein Medium zur Dichtegradienten-Zentrifugation, in dem sich ein Mittel zur Zellwandzerstörung befindet, enthält, wobei die Zentrifugation unter solchen Bedingungen durchgeführt wird, daß wengistens der Hauptanteil der Pilzzellen sich aus der Lösung in das Zentrifugationsmedium bewegt, was zur Bildung eines Überstands und eines Zentrifugationsmediums führt, das Pilzzellen und das Mittel zur Zellwandzerstörung enthält;
(b) Abtrennen des Überstands von dem Zentrifugationsmedium;
(c) Inkubieren des Zentrifugationsmediums für einen Zeitraum und bei einer Temperatur, die ausreichen, daß das Mittel zur Zellwandzerstörung die Zellwand der in dem Medium vorhandenen Pilzzellen zerstört, um Pilzzell-Protoplasten zu bilden,
(d) Vermischen des inkubierten Zentrifugationsmediums, das Pilzzell-Protoplasten enthält, mit einer Menge eines Chelatisierungsharzes; und
(e) Erhitzen der Mischung für einen Zeitraum und bei einer Temperatur, die ausreichen, die Protoplasten zu lysieren und die Pilz-DNA in einer denaturierten Form freizusetzen.

## Revendications

1. Méthode de détection de la présence d'un organisme fongique cible dans un échantillon comprenant l'étape consistant à tester ledit échantillon afin de détecter la présence ou l'absence de tout ou partie d'une molécule d'ADN double brin caractéristique de et spécifique dudit organisme, ladite molécule d'ADN comprenant une séquence de nucléotides contenant une région de bras espaceur non transcrite du motif répétitif de l'ARNr dudit organisme, dans laquelle la détection de la présence ou de l'absence de ladite molécule d'ADN caractéristique implique l'utilisation d'une sonde d'acide nucléique pouvant hydrolyser une région présélectionnée dudit motif répétitif de l'ARNr et dans laquelle l'organisme cible est *candida albicans* et dans laquelle la sonde d'acide nucléique a une séquence de nucléotides comprenant tout ou partie de la séquence de nucléotides allant du nucléotide 1 au nucléotide 936 de SEQ ID n°1 ; ou la sonde d'acide nucléique se compose d'un fragment de digestion *Pstl* de 0,9 kb dérivé du motif répétitif de l'ARNr de la souche *C. albicans* 10261 (ATCC 10261), un sous-fragment de celui-ci, ou a une séquence de nucléotides qui s'hybride avec celui-ci à une forte stringence.

2. Méthode selon la revendication 1, dans laquelle l'étape de test comprend les sous-étapes consistant à :
(a) amplifier tout ADN présent dans ledit échantillon ayant une séquence de nucléotides de tout ou partie d'une région de bras espaceur non transcrite du motif répétitif de l'ARNr des organismes cibles ;
(b) détecter la présence ou l'absence dudit ADN amplifié dans ledit échantillon.

3. Méthode selon la revendication 2, dans laquelle la procédure d'amplification utilisée est la réaction en chaîne par polymérase, la réaction par détection de ligase ou la réaction en chaîne par ligase.

4. Méthode selon la revendication 1, dans laquelle l'étape de test comprend les étapes consistant à :
(a) tester ledit échantillon avec ladite sonde, dans laquelle ladite sonde est facultativement marquée et est capable de s'hybrider avec tout ou partie d'un brin de ladite molécule d'ADN, ledit test étant réalisé dans des conditions telles ladite sonde s'hybride avec tout ADN contenant une région de bras espaceur non transcrite du motif répétitif d'ARNr de l'organisme cible qui est présent dans l'échantillon pour former un ADN hybride ;
(b) détecter la présence ou l'absence dudit ADN hybride dans l'échantillon.

5. Méthode selon la revendication 4, dans laquelle le test est effectué dans des conditions d'hybridation à basse stringence.

6. Méthode selon l'une quelconque des revendications 1 à 5, dans laquelle ledit échantillon contient de l'ADN extrait du sang ou de tissus.

7. Méthode pour détecter la présence d'un ou de plusieurs organismes fongiques cibles d'un groupe dans un échantillon comprenant les étapes consistant à :
(a) tester ledit échantillon pour détecter la présence de tout ou partie d'une molécule d'ADN double brin caractéristique de et spécifique audit organisme, ladite molécule d'ADN comprenant une séquence de nucléotides codant pour le gène d'ARNr 5S de l'un desdits organismes ; et
(b) tester séparément ledit échantillon pour détecter la présence d'une ou plusieurs molécules d'ADN double brin d'un groupe ou une partie de celles-ci, chaque dite molécule d'ADN double brin étant caractéristique et spécifique à un organisme cible différent et comprenant une séquence de nucléotides d'une région de bras espaceur non transcrite du motif répétitif d'ARNr de cet organisme, ledit test comprenant l'utilisation d'une méthode selon l'une quelconque des revendications 1 à 6.

8. Méthode selon la revendication 7, dans laquelle l'étape de test (a) comprend les sous-étapes consistant à :
(i) amplifier tout ADN présent dans ledit échantillon ayant une séquence de nucléotides du gène d'ARNr 5S d'un organisme cible ; et
(ii) détecter la présence ou l'absence de tout ARN amplifié dans ledit échantillon.

9. Méthode selon la revendication 7 ou la revendication 8, dans laquelle l'étape de test (b) comprend les sous-étapes consistant à :
(i) amplifier n'importe lequel desdits groupes de molécules d'ADN présentes dans ledit échantillon ; et
(ii) détecter la présence ou l'absence de n'importe lequel desdits groupes de molécules d'ADN dans des quantités amplifiées.

10. Méthode selon l'une quelconque des revendications 7 à 9, dans laquelle ledit échantillon contient de l'ADN libéré par les étapes consistant à :
(a) centrifuger un réceptacle contenant une solution de cellules fongiques et un milieu de centrifugation en gradient de densité incorporant un perturbateur des parois cellulaires, ladite centrifugation étant réalisée dans des conditions telles qu'au moins la majeure partie de cellules fongiques migrent hors de la solution dans ledit milieu de centrifugation, ce qui entraîne la formation d'un surnageant et d'un milieu de centrifugation contenant des cellules fongiques et ledit perturbateur des parois cellulaires ;
(b) séparer ledit surnageant et ledit milieu de centrifugation ;
(c) incuber ledit milieu de centrifugation pendant une certaine période de temps et à une température suffisante pour que ledit perturbateur des parois cellulaires perturbe la paroi cellulaire des cellules fongiques présente dans ledit milieu pour former des protoplastes de cellules fongiques ;
(d) mélanger ledit milieu de centrifugation incubé contenant les protoplastes de cellules fongiques et une certaine quantité de résine chélatante ; et
(e) chauffer ledit mélange pendant une période de temps et à une température suffisantes pour lyser lesdits protoplastes et pour libérer l'ADN fongique sous une forme dénaturée.
